# EUROPEAN PATENT APPLICATION

(11) **EP 2 705 856 A1**
(43) Date of publication of application: **12.03.2014**
(21) Application number: 12183510.2
(22) Date of filing: 07.09.2012
(51) Int. Cl.: A61K 47/48, A61P 37/06

(54) **Compounds for the treatment of neurodegenerative disorders**

(71) Applicant: Deutsches Zentrum für Neurodegenerative Erkrankungen e.V., 53175 Bonn (DE)
(72) Inventor: Striggow, Frank, 39175 Gerwisch (DE); Schmidt, Werner, 17268 Boitzenburger Land (DE); Fischer, Gunter, 06120 Halle (DE); Theuerkorn, Martin, 06108 Halle (DE); Malesevic, Miroslav, 06108 Halle (DE); Weiwad, Matthias, 06120 Halle (DE); Prell, Erik, 06249 Mücheln (DE)
(74) Representative: Kalhammer, Georg

(57) **Abstract**

The present invention relates to compounds and compositions useful in the treatment and/or prevention of neurodegenerative diseases, in particular multiple sclerosis (MS).

## Description

### BACKGROUND OF THE INVENTION

The present invention pertains to compounds and compositions useful in the treatment and/or prevention of neurodegenerative diseases, in particular multiple sclerosis (MS).

MS is an inflammatory disease of the central nervous system (CNS) in which demyelination as well as axonal injury and neuronal cell degeneration result in permanent neurologic disability. Demyelination is caused by autoimmune attacks directed against oligodendrocytes, the cellular constituents of axonal myelin shields in the CNS. Oligodendrocyte degeneration and axonal unshielding is followed by axonal degeneration and neuronal cell loss - major contributors to permanent neurologic injury. In addition, progressive axonal and neuronal cell degeneration may be critical to the development of secondary progressive MS. Pharmacologic agents that can protect against oligodendrocyte and/or neuronal degeneration and/or promote axonal and neuronal regeneration may thus be useful for preventing progressive disability in MS.

FK506 (tacrolimus) is an approved immunosupressant macrolide drug isolated from *streptomyces tsukubaensis* and used for the prevention of allograft rejection after liver or kidney transplantation. FK506 exerts its immunosuppressive effects by binding to FK506 binding protein-12 (FKBP-12) in lymphocytes and inhibiting calcineurin activation, resulting in a marked reduction in interleukin (IL-2) production. FK506 also has neuroprotective/neurodegenerative effects and is the prototype of a novel class of drugs, neuroimmunophilin ligands. Gold et al. (2004), Journal of Neuroscience Research 77:367-377 describe that FK506 markedly protects against demyelination and axonal loss in experimental autoimmune encephalomyelitis, an animal model of MS.

It is further known that the compound *N*-(*N*,*N*-Dimethylcarboxamidomethyl)cycloheximid) (DM-CHX) inhibits the protein FKBP38 and exhibits neuroprotective and neuroregenerative properties in a rat model of transient focal cerebral ischemia (Edlich et al. (2006) The Journal of Biological Chemistry Vol. 281, No. 21, 14961-14970).

The "FK506 binding proteins" (FKBPs) display an FK506-inhibitable peptidyl-prolyl cis-trans isomerase (PPlase) activity. FKBPs catalyze the interconversion of cis-trans isomers of X-Pro peptide bonds and thereby, are crucially involved in the regulation of protein structure and function. FKBPs are a subfamily of the immunophilins, a family of highly conserved proteins with a peptidyl-prolyl isomerase activity that binds certain immunosuppressive drugs.

Besides FKBPs, another immunophilin subfamily has PPIase activity: The cyclophilins. Cyclosporin A (CsA) is a cyclic peptide initially isolated from the fungus *tolypocladium inflatum.* CsA binds to a cyclophilin and inhibits its PPlase activity. In analogy to the complex of FK506/FKBP, also the CsA/cyclophilin complex blocks the protein phosphatase calcineurin, consequently leading to immunosuppression.

While the neuroprotective and neuroregenerative effects of FKBP inhibitors and cyclophilin inhibitors are promising, their immunosuppressive action leads to significant side effects. Therefore, administration of the compounds bears the risk of immunosuppression thus making the patients susceptible to infections.

The present invention therefore aims to provide compounds and compositions with reduced immunosuppressive effects while retaining neuroprotective and/or neuroregenerative properties.

### SUMMARY OF THE INVENTION

The inventors found that certain bifunctional molecules comprising an FKBP inhibitor moiety and a cyclosporine moiety exhibit reduced immunosuppressive activity while still being capable of inhibiting immunophilins. The present invention therefore relates to the following subject matter (1) to (16).
(1) A compound comprising an FKBP inhibitor covalently linked to the amino acid residue at position 8 of a cyclosporine.
(2) The compound of (1), represented by the structural formula Cs-L-F, wherein
   Cs is the cyclosporine,
   L is a linker, and
   F is the FKBP inhibitor.
(3) The compound of (1) or (2), wherein the cyclosporine is Cyclosporin A or a derivative thereof, in particular [D-Ser]⁸CsA.
(4) The compound of any one of (1) to (3), wherein the linker is an optionally substituted aliphatic, alicyclic, heteroaliphatic, heterocyclic, aryl, arylalkyl, or heteroaromatic group and consists of 5 to 300 atoms.
(5) The compound of (4), wherein linker is a C₁₋₂₀, saturated or unsaturated, straight or branched, hydrocarbon chain, wherein one or more methylene units of the linker are optionally and independently replaced by phenylene, piperazinylene, piperidinylene, pyridinylene, morpholinylene, 1,4-dioxanylene, pyrrolylene, furanylene, thiophenylene, thiazolylene, oxazolylene, imidazolylene, -N=N-, -N(R)-, -N(R)C(O)N(R)-, -N(R)C(O)O-, -OC(O)N(R)-, -N(R)C(O)-, -C(O)N(R)-, -N(R)SO₂-, - SO₂N(R)-, -O-, -C(O)-, -OC(O)-, -C(O)O-, -S-, -SO-, -SO₂-, -C(=S)-, or -C(=NR)-; wherein R is selected from the group consisting of hydrogen and C₁₋₆ alkyl.
(6) The compound of any one of (1) to (5), wherein the FKBP inhibitor is the compound FK506.
(7) The compound of any one of (1) to (5), wherein the FKBP inhibitor is CHX.
(8) The compound of (6), which is 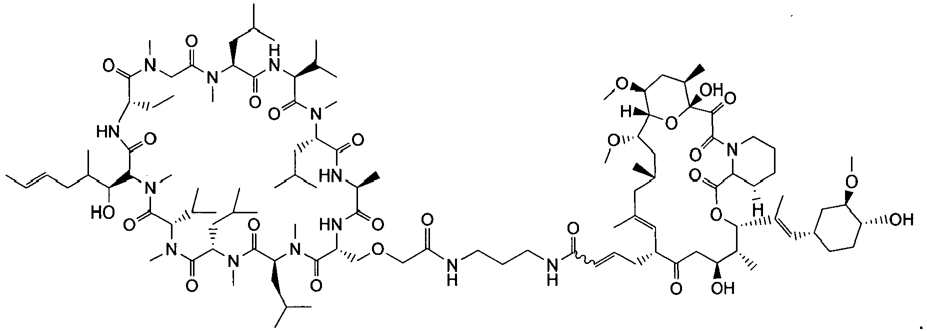
(9) The compound of (7), which is 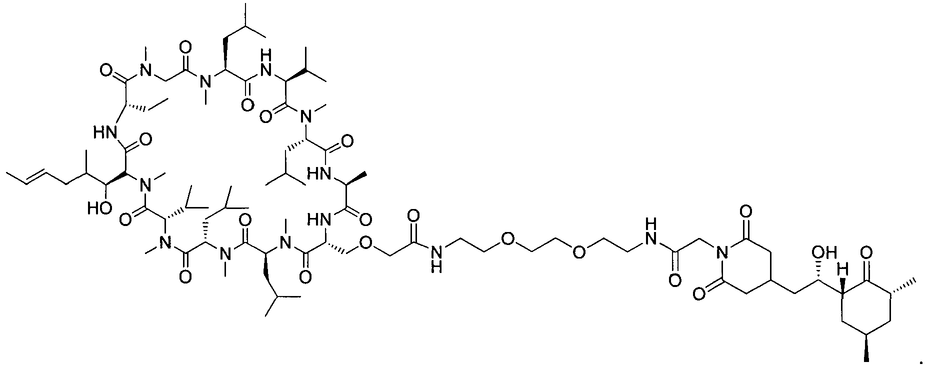
(10) The compound of any one of (1) to (9) which is capable of reducing the clinical signs of experimental autoimmune encephalomyelitis (EAE) in a mouse MS model.
(11) The compound of any one of (1) to (10) which is capable of inhibiting at least one FKBP and at least one cyclophilin.
(12) The compound of any one of (1) to (11) having a lower immunosuppressive effect than a mixture of the cyclosporine and the FKBP inhibitor, as determined in a calcineurin inhibition assay.
(13) A pharmaceutical composition comprising a compound according to any one of (1) to (12) or a pharmaceutically acceptable salt, ester, or solvate thereof, and a pharmaceutically acceptable excipient.
(14) A compound comprising an FKBP inhibitor covalently linked to a cyclosporine for use in the treatment of a neurodegenerative disorder.
(15) The compound for use according to (14), wherein said neurodegenerative disorder is selected from the group consisting of multiple sclerosis, Alzheimer's disease, Parkinson's disease, cerebral ischemia, Huntington's disease and amyotrophic lateral sclerosis.
(16) The compound for use according to (14) or (15), which is a compound as defined in any one of (1) to (13).

### DETAILED DESCRIPTION

The present invention relates to certain compounds that are useful in the treatment and/or prevention of neurodegenerative disorders such as MS.

The compounds of the present invention comprise (i) at least one FKBP inhibitor and (ii) at least one cyclosporine. These two elements (i) and (ii) are covalently linked wherein the FKBP inhibitor is bound to the amino acid residue at position 8 of the cyclosporine.

FK506 binding proteins (FKBPs) are polypeptides that display an FK506-inhibitable PPlase activity, i.e. they are able to accelerate the interconversion of cis-trans isomers of X-Pro peptide bonds. The FKBP family is divided into at least four different groups based on the intracellular localization and domain composition (Rulten et al. (2006) Mamm Genome 17:322-331). The four groups are a cytoplasmic group (FKBP12 and 12.6), a nuclear group (FKBP25 and FKBP135), a tetratricopeptide repeat (TPR)-containing group (FKBP36, FKBP37, FKBP38, FKBP51, and FKBP52), and a secretory pathway group (FKBP13, FKBP19, FKBP22, FKBP23, FKBP60 and FKBP65), see Gerard et al. (2011) Mol. Neurobiol. 44:13-27 and references cited therein.

The term "FKBP inhibitor" refers to a compound or composition which is capable of inhibiting the biological activity of at least one FKBP. The biological activity is preferably the enzymatic cis-trans-peptidyl-prolyl isomerase activity of the FKBP. Peptidyl prolyl isomerase activity can be evaluated by known methods described in the literature (G. Fischer, H. Bang, B. Ludwig, K. Mann, J. Hacker, Mol. Microbiol. 1992, 6, 1375-1383). A suitable test to determine the enzymatic cis-trans-peptidyl-prolyl isomerase activity is described in Example 1 herein below.

In one embodiment, the FKBP inhibitor is an inhibitor of FKBP12. In another embodiment, the FKBP inhibitor is an inhibitor of FKBP38. In yet another embodiment, the FKBP inhibitor is an inhibitor of FKBP52.

FKBP inhibitors in accordance with the present invention include, but are not limited to, the FKBP inhibitors disclosed in U.S. Pat. No. 6,291,510, U.S. Pat. No. 6,140,357, U.S. Pat. No. 6,509,477, U.S. Pat. No. 6,509,477, or U.S. Pat. No.6,509,477); the FKBP-binding pipecolic acid derivatives (U.S. Pat. No. 6,500,843, U.S. Pat. No. 6,022,878, U.S. Pat. No. 5,846,981, U.S. Pat. No. 5,843,960, U.S. Pat. No. 5,801,197); and the FKBP inhibitors disclosed in U.S. Pat. No. 6,509,464, U.S. Pat. No. 6,495,549, or U.S. Pat. No. 6,166,0111. These compound are incorporated herein by reference.

Preferred FKPB inhbitors are FK506, DM-CHX rapamycin, GPI-1046, GPI-1485, and V-10,367.

The structure of FK506 is shown in Formula (I):

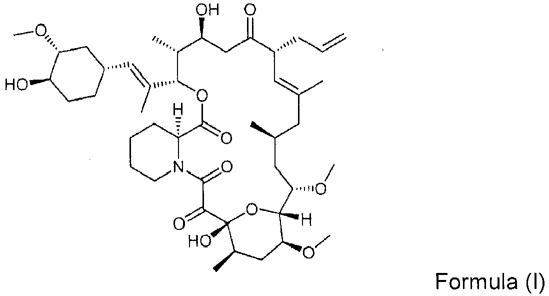

Where FK506 is used, variants or analogs of FK506 are included, such as rapamycin, pimecrolimus, or synthetic ligands of FK506 binding proteins (SLFs) such as those disclosed in U.S. Pat. Nos. 5,665,774, 5,622,970, 5,516,797, 5,614,547, and 5,403,833 or described by Holt et al., "Structure-Activity Studies of Synthetic FKBP Ligands as Peptidyl-Prolyl Isomerase Inhibitors," Bioorganic and Medicinal Chemistry Letters, 4(2):315-320 (1994).

The structure of DM-CHX is depticted in Formula (II):

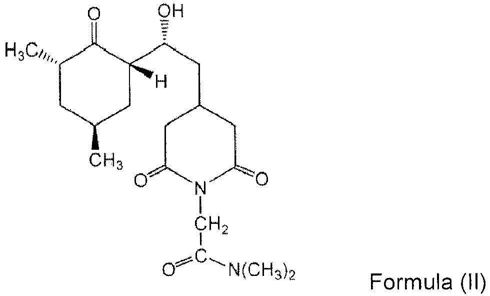

It is to be understood that the FKBP inhibitors comprised in the compounds of the present invention are chemically modified due to the covalent coupling to the cyclosporine. For example, the Carboxymethyl cycloheximide moiety may be linked to the cyclosporine via its carboxy group. The reference to "FKBP inhibitors" as used herein includes any modified forms of the FKBP inhibitors mentioned *supra,* where the modification is due to the linkage to the cyclosporine.

The cyclosporin in accordance with this invention may be any cyclosporin or cyclosporin analogue which is capable of inhibiting the PPlase activity of a cyclophilin. Examples include the following.
- Cyclosporin analogues for therapeutically influencing the immune and respiratory systems (U.S. Pat. No. 7,226,906, U.S. Pat. No. 7,141,648, U.S. Pat. No. 6,927,208, U.S. Pat. No. 5,994,299; U.S. Pat. No. 5,977,067, U.S. Pat. No. 5,965,527, U.S. Pat. No. 4,288,431, U.S. Pat. No. 6,455,518, U.S. Pat. No. 6,432,968, U.S. Pat. No. 6,046,328).
- Cyclosporin alkines (U.S. Pat. No. 7,378,391, U.S. Pat. No. 7,361,636).
- 3-ethers and 3-thioethers of cyclosporin (U.S. Pat. No. 7,196,161).
- 3-position cyclosporin derivatives (U.S. Pat. No. 6,987,090, U.S. Pat. No. 6,790,830, U.S. Pat. No. 6,762,164).
- Cyclosporin analogues (U.S. Pat. No. 6,809,077).
- Cyclosporin derivatives, particularly suitable for the treatment of rheumatoid arthritis (U.S. Pat. No. 6,770,279).
- Cyclosporin derivatives (U.S. Pat. No. 5,948,884).
- 8-position cyclosporin derivatives (U.S. Pat. No. 5,318,901).
- Cyclosporin peptolides (U.S. Pat. No. 5,116,816), particularly suitable as immunosuppressive, anti-inflammatory and anti-parasitic active ingredients.
- Cyclosporin analogues with modified C-9 amino acids (U.S. Pat. No. 4,885,276; U.S. Pat. No. 4,798,823) which have immunosuppressive properties.

Preferably, the cyclosporine is cyclosprin A which has the following formula:

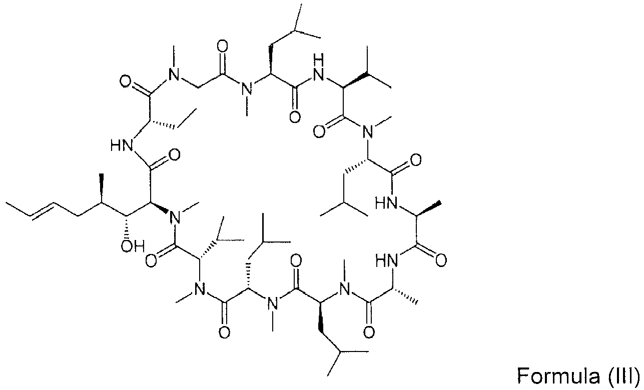

According to the present invention the cyclosporin is linked to the FKBP inhibitor via its amino acid at position 8. In the native structure of cyclosporin this is D-alanine. According to a preferred embodiment, the cyclosporin is [D-Ser⁸]-CsA.

It is to be understood that the cyclosporins comprised in the compounds of the present invention are chemically modified due to the covalent coupling to the cyclosporine. For example, the hydroxyl group of [D-Ser⁸]-CsA may be converted into a group -O-R, where R is an alkylene group or another aliphatic or aromatic group. The reference to "cyclosporin" as used herein includes any modified forms of the cyclosporins mentioned *supra,* where the modification is due to the linkage to the FKBP inhibitor.

Typically, the FKBP inhibitor and the cyclosporine are covalently linked to each other via a linker or spacer. The terms linker and spacer are used interchangeably herein.

The linker is an optionally substituted aliphatic, alicyclic, heteroaliphatic, heterocyclic, aromatic, heteroaromatic or arylalkyl substituent. The linker usually comprises 2 to 200 carbon atoms, preferably 4 to 100 carbon atoms, more preferably 5 to 50 carbon atoms, most preferably 5 to 10 carbon atoms. The linker typically consists of 5 to 500 atoms, preferably of 8 to 200 atoms, most preferably of 10 to 100 atoms.

The term "aliphatic substituent", as used herein, includes saturated or unsaturated, branched or unbranched aliphatic bivalent substituents. In the present application aliphatic substituent is intended to include, but is not limited to alkylene, alkenylene, alkynylene and alkadienylene substituents.

The term "alkylene" used is the present application relates a saturated branched or unbranched aliphatic bivalent substituent.

The term "alkenylene" as used is the present application is an unsaturated branched or unbranched aliphatic bivalent substituent having a double bond between two adjacent carbon atoms.

The term "alkynylene" as used is the present application is an unsaturated branched or unbranched aliphatic bivalent substituent having a tripple bond between two adjacent carbon atoms.

The term "alkadienylene" as used is the present application is an unsaturated branched or unbranched aliphatic bivalent substituent having two double bonds between two adjacent carbon atoms.

As used herein, the term "alicyclic" refers to a monocyclic, bicyclic, or tricyclic substituent, which may be saturated or partially saturated, i.e. possesses one or more double bonds. Monocyclic substituents are exemplified by a saturated cyclic hydrocarbon group containing from 3 to 8 carbon atoms. Examples of monocyclic cycloalkyl substituents include cyclopropyl, cyclobutyl, cyclopentyl, cyclopentenyl, cyclohexyl, cyclohexenyl, cycloheptyl and cyclooctyl. Bicyclic fused cycloalkyl substituents are exemplified by a cycloalkyl ring fused to another cycloalkyl ring. Examples of bicyclic cycloalkyl substituents include, but are not limited to decalin, 1,2,3,7,8,8a-hexahydro-naphthalene, and the like. Tricyclic cycloalkyl substituents are exemplified by a cycloalkyl bicyclic fused ring fused to an additional cycloalkyl substituent.

The term "heteroaliphatic substituent", as used herein, refers to a bivalent substituent, in which one or more carbon atoms have been substituted with a heteroatom, for instance, with an oxygen, sulfur, nitrogen, phosphorus or silicon atom, wherein the nitrogen and sulfur atoms may optionally be oxidized, and the nitrogen heteroatom may optionally be quaternized. The heteroatom(s) O, N and S may be placed at any interior position of the heteroaliphatic substituent. Examples include -CH₂-CH₂-O-CH₂-, -CH₂-CH₂-NH-CH₂-, - CH₂-CH₂-N(CH₃)-CH₂-, -CH₂-S-CH₂-CH₂-, -S(O)-CH₂-, -CH₂-CH₂-S(O)₂-CH₂-, -CH=CH-O-CH₂-, -CH₂-CH=N-OCH₂-, and -CH=CH-N(CH₃)-CH₂-. A heteroaliphatic substituent may be linear or branched, and saturated or unsaturated.

As used herein, "aromatic substituent" is intended to mean any stable monocyclic, bicyclic or polycyclic carbon ring of up to 10 atoms in each ring, wherein at least one ring is aromatic, and may be unsubstituted or substituted. Examples of such aromatic substituents include phenyl, p-toluenyl (4-methylphenyl), naphthyl, tetrahydronaphthyl, indanyl, biphenyl, phenanthryl, anthryl or acenaphthyl. In cases where the aromatic substituent is bicyclic and one ring is non-aromatic, it is understood that attachment is via the aromatic ring.

The term "arylalkyl substituents" refers to alkyl substituents as described above wherein one or more bonds to hydrogen contained therein are replaced by a bond to an aryl substituent as described above. It is understood that an arylalkyl substituents is connected to the carbonyl group if the compound of Formula I through a bond from the alkyl substituent. Examples of arylalkyl substituents include, but are not limited to, benzyl (phenylmethyl), p-trifluoromethylbenzyl (4-trifluoromethylphenylmethyl), 1-phenylethyl, 2-phenylethyl, 3-phenylpropyl, 2-phenylpropyl and the like.

The term "heteroaromatic substituent" as used herein, represents a stable monocyclic, bicyclic or polycyclic ring of up to 10 atoms in each ring, wherein at least one ring is aromatic and contains from 1 to 4 heteroatoms selected from the group consisting of O, N and S. Bicyclic heteroaromatic substituents include phenyl, pyridine, pyrimidine or pyridazine rings that are
a) fused to a 6-membered aromatic (unsaturated) heterocyclic ring having one nitrogen atom;
b) fused to a 5- or 6-membered aromatic (unsaturated) heterocyclic ring having two nitrogen atoms;
c) fused to a 5-membered aromatic (unsaturated) heterocyclic ring having one nitrogen atom together with either one oxygen or one sulfur atom; or
d) fused to a 5-membered aromatic (unsaturated) heterocyclic ring having one heteroatom selected from O, N or S.

Heteroaryl groups within the scope of this definition include but are not limited to: benzoimidazolyl, benzofuranyl, benzofurazanyl, benzopyrazolyl, benzotriazolyl, benzothiophenyl, benzoxazolyl, carbazolyl, carbolinyl, cinnolinyl, furanyl, indolinyl, indolyl, indolazinyl, indazolyl, isobenzofuranyl, isoindolyl, isoquinolyl, isothiazolyl, isoxazolyl, naphthpyridinyl, oxadiazolyl, oxazolyl, oxazoline, isoxazoline, oxetanyl, pyranyl, pyrazinyl, pyrazolyl, pyridazinyl, pyridopyridinyl, pyridazinyl, pyridyl, pyrimidyl, pyrrolyl, quinazolinyl, quinolyl, quinoxalinyl, tetrazolyl, , tetrazolopyridyl, thiadiazolyl, thiazolyl, thienyl, triazolyl, azetidinyl, aziridinyl, 1,4-dioxanyl, hexahydroazepinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylenedioxybenzoyl, tetrahydrofuranyl, tetrahydrothienyl, acridinyl, carbazolyl, cinnolinyl, quinoxalinyl, pyrrazolyl, indolyl, benzotriazolyl, benzothiazolyl, benzoxazolyl, isoxazolyl, isothiazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, tetra-hydroquinoline. In cases where the heteroaryl substituent is bicyclic and one ring is non-aromatic or contains no heteroatoms, it is understood that attachment is via the aromatic ring or via the heteroatom containing ring, respectively. If the heteroaryl contains nitrogen atoms, it is understood that the corresponding N-oxides thereof are also encompassed by this definition.

The aliphatic, heteroaliphatic, aromatic and heteroaromatic substituents can be optionally substituted one or more times, the same way or differently with any one or more of the following substituents including, but not limited to: aliphatic, heteroaliphatic, aromatic and heteroaromatic substituents, aryl, heteroaryl; alkylaryl; heteroalkylaryl; alkylheteroaryl; heteroalkylheteroaryl; alkoxy; aryloxy; heteroalkoxy; heteroaryloxy; alkylthio; arylthio; heteroalkylthio; heteroarylthio; F; Cl; Br; I; -OH; -NO₂; -CN; -CF₃; -CH₂CF₃; -CHCl₂; -CH₂OH; -CH₂CH₂OH; -CH₂NH₂; -CH₂SO₂CH₃; -C(O)Rₓ; -CO₂(Rₓ); -CON(Rₓ)₂; -OC(O)Rₓ; -OCO₂Rₓ; -OCON(Rₓ)₂; -N(Rₓ)₂; -S(O)Rₓ; -S(O)₂Rₓ; -NRₓ(CO)Rₓ wherein each occurrence of Rₓ independently includes, but is not limited to, aliphatic, alicyclic, heteroaliphatic, heterocyclic, aromatic, heteroaromatic, aryl, heteroaryl, alkylaryl, alkylheteroaryl, heteroalkylaryl or heteroalkylheteroaryl, wherein any of the aliphatic, alicyclic, heteroaliphatic, heterocyclic, alkylaryl, or alkylheteroaryl substituents described above and herein may be substituted or unsubstituted, branched or unbranched, saturated or unsaturated, and wherein any of the aromatic, heteroaromatic, aryl, heteroaryl, -(alkyl)aryl or -(alkyl)heteroaryl substituents described above and herein may be substituted or unsubstituted. Additionally, it will be appreciated, that any two adjacent substituents taken together may represent a 4, 5, 6, or 7-membered substituted or unsubstituted alicyclic or heterocyclic substituents. Additional examples of generally applicable substituents are illustrated by the specific embodiments shown below.

In certain embodiments, the linker is a C₁₋₂₀, preferably a C₃₋₁₈, more preferably a C₅₋₁₆, most preferably a C₈₋₁₅, saturated or unsaturated, straight or branched, hydrocarbon chain, wherein one or more methylene units of the linker are optionally and independently replaced by phenylene, piperazinylene, piperidinylene, pyridinylene, morpholinylene, 1,4-dioxanylene, pyrrolylene, furanylene, thiophenylene, thiazolylene, oxazolylene, imidazolylene, -N=N-, -N(R)-, -N(R)C(O)N(R)-, -N(R)C(O)O-, -OC(O)N(R)-, -N(R)C(O)-, - C(O)N(R)-, -N(R)SO₂-, -SO₂N(R)-, -O-, -C(O)-, -OC(O)-, -C(O)O-, -S-, -SO-, -SO₂-, -C(=S)-, or -C(=NR)-; wherein R is selected from the group consisting of hydrogen and C₁₋₆ alkyl.

The term "alkyl" used is the present application relates a saturated branched or unbranched aliphatic univalent substituent. Preferably, the alkyl substituent has 1 to 22 carbon atoms, more preferred 1 to 6 carbon atoms, even more preferred 1 to 3 carbon atoms. Accordingly, examples of the alkyl substituent include methyl, ethyl, *n*-propyl, isopropyl, *n*-butyl, isobutyl, *sec*-butyl, *tert*-butyl, *n*-pentyl and *n*-hexyl and preferable examples include methyl, ethyl, n-propyl and isopropyl, whereby ethyl and isopropyl are particularly preferred.

In one embodiment, the compound of the present invention has the structure Cs-L-F, wherein Cs is the cyclosporine, L is a linker, and F is the FKBP inhibitor.

The Cs is preferably a cyclosporine as defined *supra.* The FKBP is preferably an FKPB as defined *supra.* The linker L is preferably a linker as defined *supra.*

According to a preferred embodiment, the Cs is 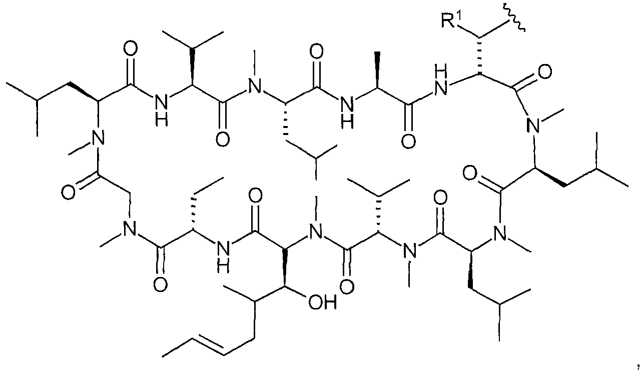 wherein R1 is selected from the group consisting of hydrogen, hydroxyl, C₁₋₆ alkyl, amino, and halogen.

It is further preferred that F is 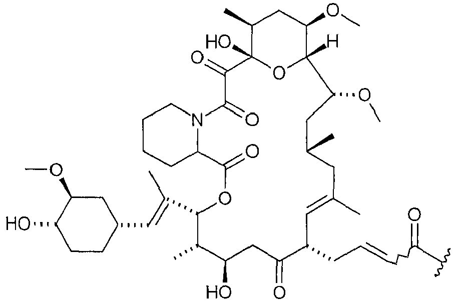 or

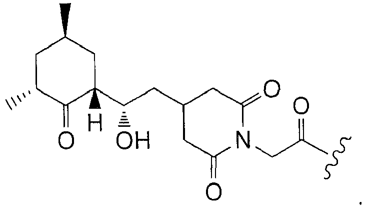

In a preferred embodiment, Cs is 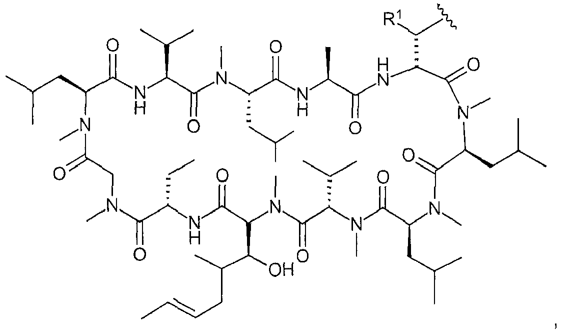 wherein R¹ is as defined above, and F is

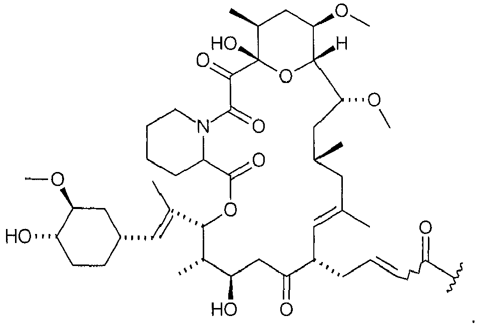

In another preferred embodiment, Cs is 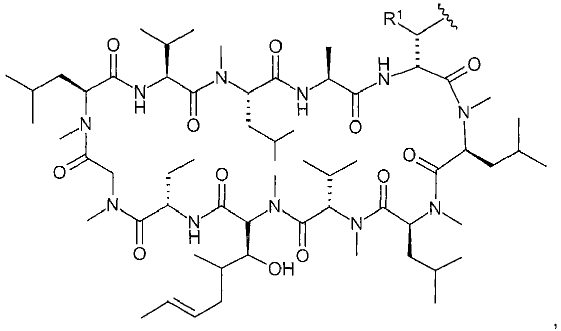 wherein R¹ is as defined above, and F is

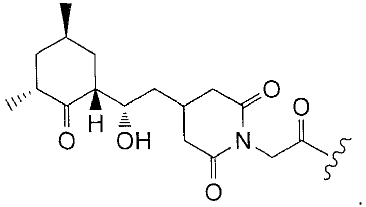

The linker L is preferably selected from the group consisting of 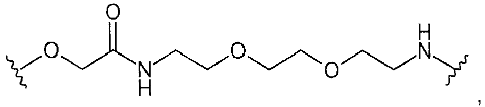 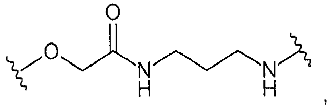 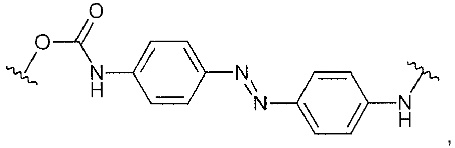 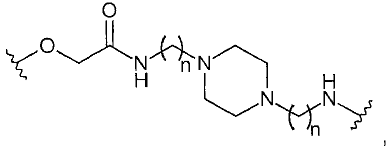 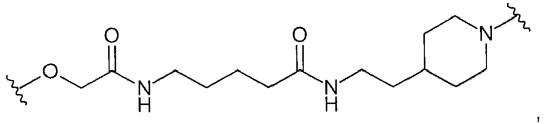 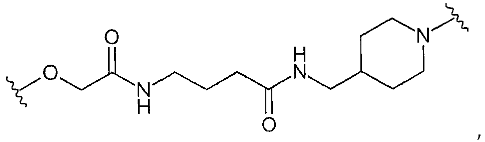 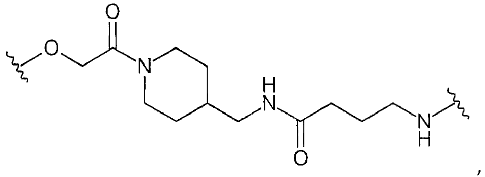 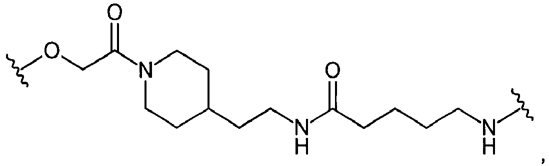 and 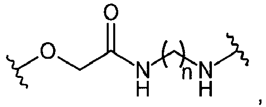 wherein n is an integer from 1 to 20, preferably from 2 to 10, more preferably from 3 to 8, most preferably from 4 to 6. In a specific embodiment, n is 1, 3, 5, 7 or 9. The left-hand end of the linkers depicted above is typically linked to the cyclosporine, whereas the righthand end is typically bound to the FKBP inhibitor.

Preferred compounds according to the present invention include, but are not limited to, the following compounds: 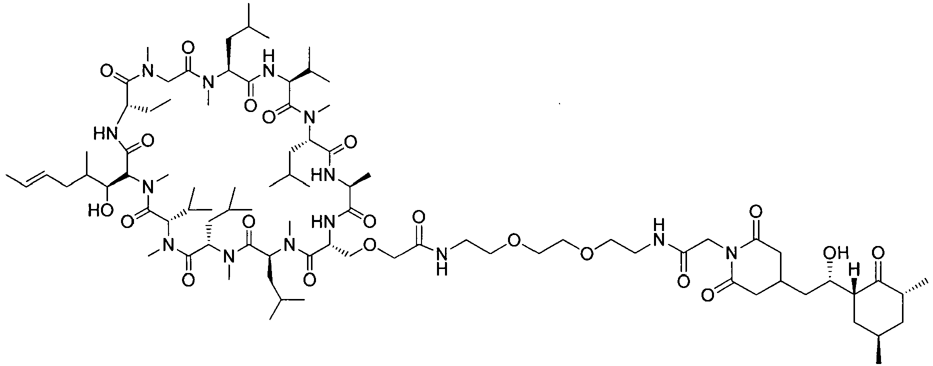 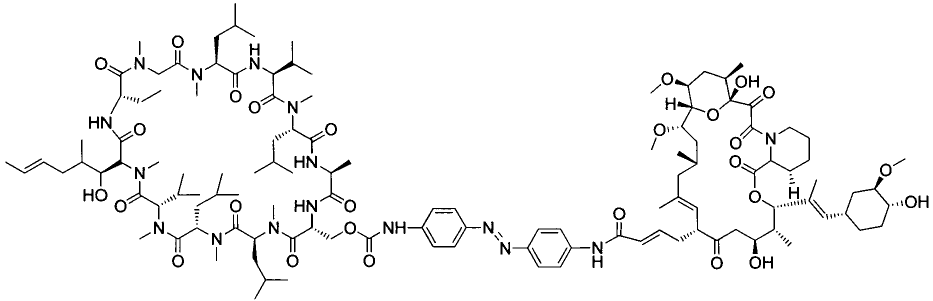 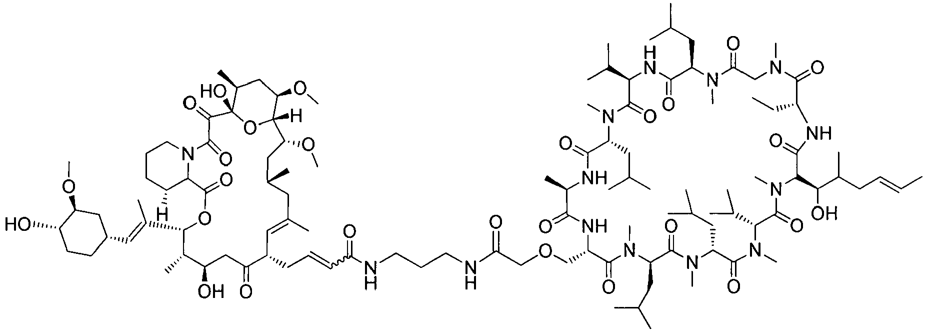 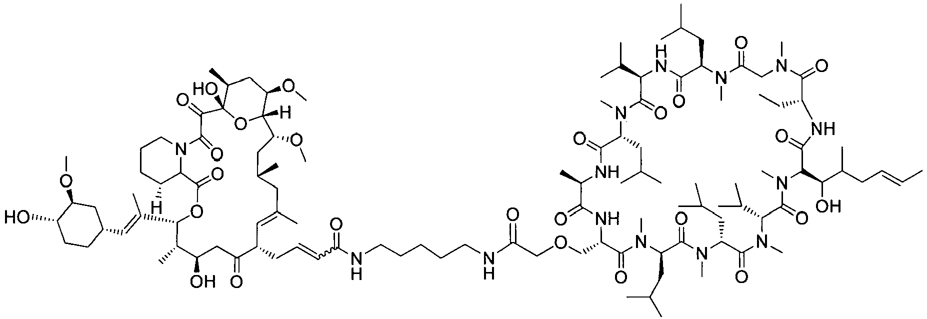 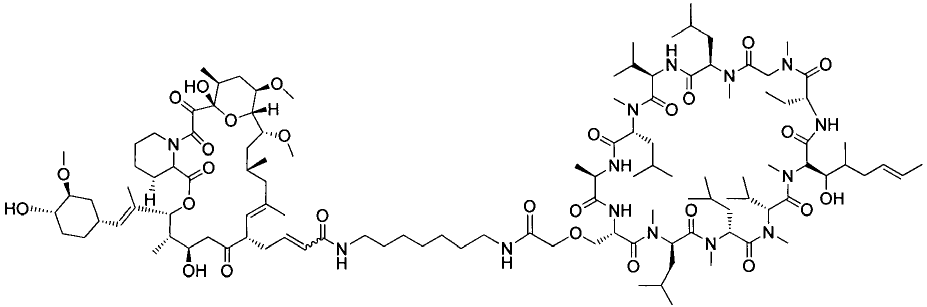 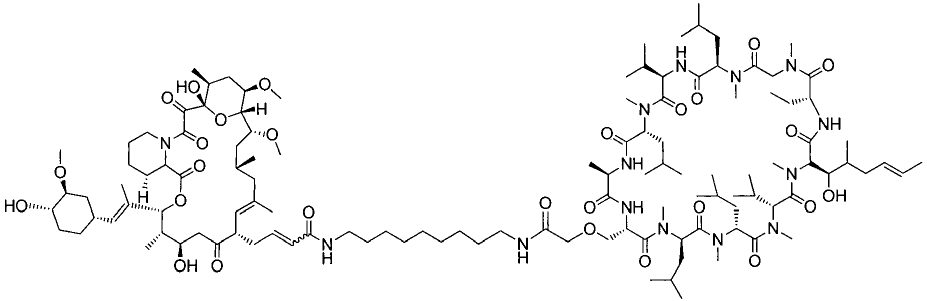 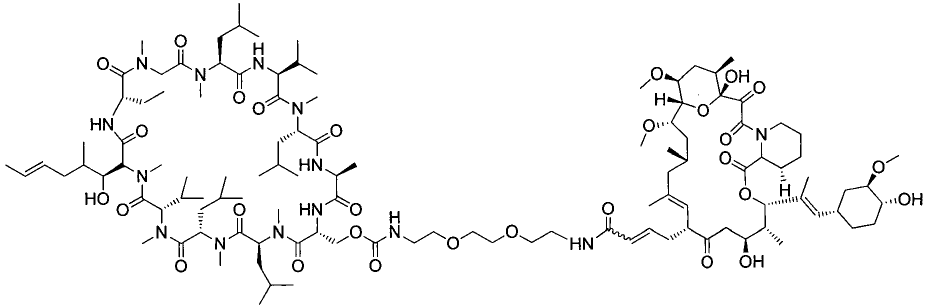 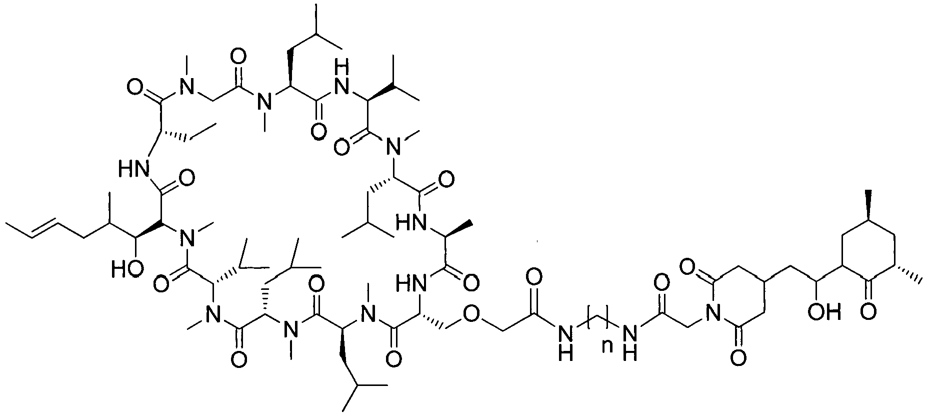 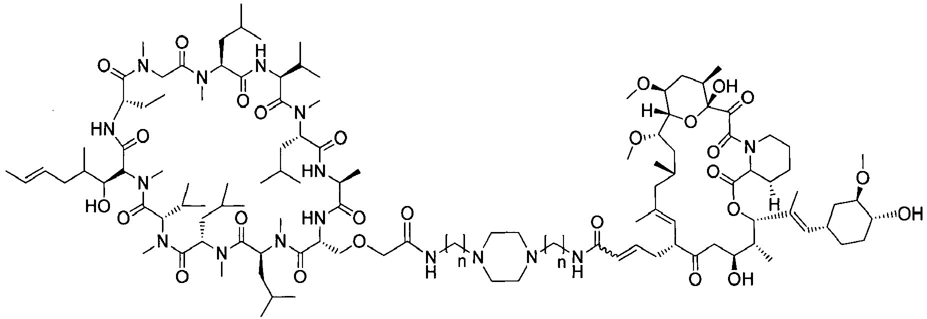 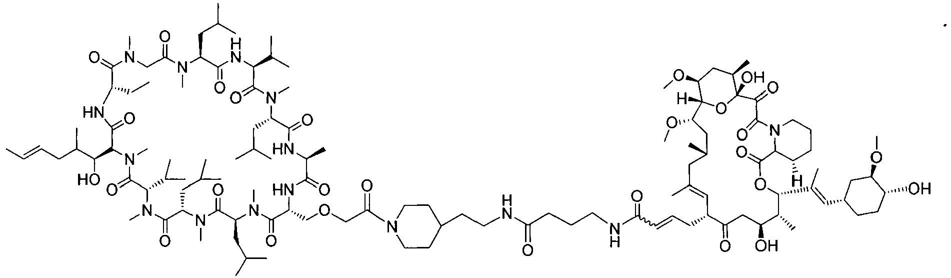 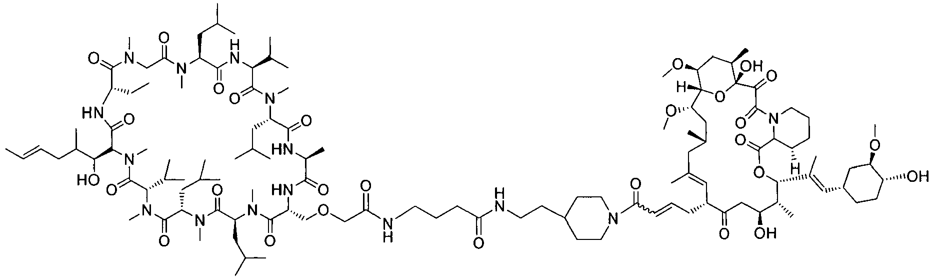 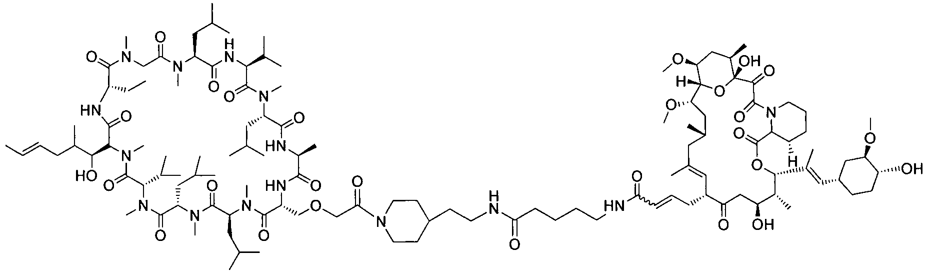 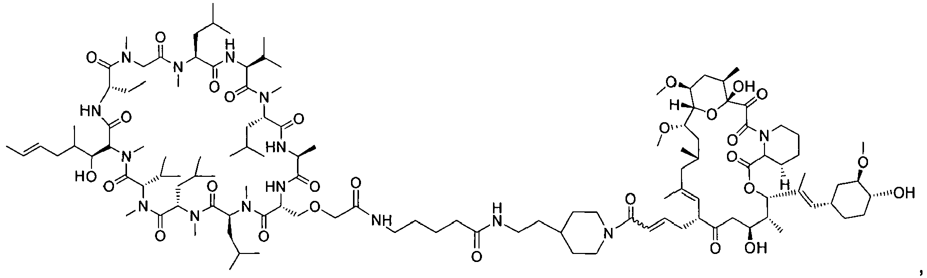 wherein n is an integer from 1 to 20, preferably from 2 to 10, more preferably from 3 to 8, most preferably from 4 to 6. In a specific embodiment, n is 1, 3, 5 or 7.

The compounds of the present invention have PPlase inhibiting activity. In one embodiment, the compounds of the invention are capable of inhibiting at least one FKBP, preferably FKBP12. In another embodiment, the compounds of the invention are capable of inhibiting at least one cyclophilin, preferably hCypA. The IC50 value of the compound of the invention, as determined in a protease coupled PPlase assay as defined in Example 1, is preferably less than 100 µM, more preferably less than 75 µM, most preferably less than 50 µM, for hFKBP12 and/or hCypA.

The compounds of the invention are further capable of reducing the clinical signs of experimental autoimmune encephalomyelitis (EAE) in a mouse model as defined in Example 2 hereinbelow.

The compounds of the present invention preferably have a reduced immunosuppressive activity as compared to the "free mixture" of cyclosporine and FKBP inhibitor, i.e. a mixture of cyclosporine and FKBP inhibitor wherein the two components are not covalently linked. The reduction is preferably at least 10%, more preferably at least 20%, more preferably at least 30%, more preferably at least 40%, more preferably at least 50%. The immunosuppressive activity is determined by a calcineurin inhibition assay as defined in the examples or by an NFAT reporter gene assay as defined in the examples (for Calcineurin, see also Cell; 1991,66,807-815; for NFAT, see also J. Biol. Chem.;2004,2792470-2479).

A further aspect of the present invention is a pharmaceutical composition comprising a compound of the present invention or a pharmaceutically acceptable salt thereof. Compounds of the present invention may be combined with a pharmaceutically acceptable carrier to form a pharmaceutical composition. Remington's Pharmaceutical Sciences, Sixteenth Edition, E. W. Martin (Mack Publishing Co., Easton, Pa., 1980) discloses various carriers used in formulating pharmaceutical compositions and known techniques for the preparation thereof. In certain embodiments, the pharmaceutical composition includes a pharmaceutically acceptable amount of an inventive compound. The amount of active ingredient which can be combined with a carrier material to produce a single dosage form will vary depending upon the individual being treated, and the particular mode of administration. The amount of active ingredient that can be combined with a carrier material to produce a single dosage form will generally be that amount of the compound which produces a therapeutic effect.

The pharmaceutical composition of the invention may further contain at least one further active ingredient useful in the treatment of neurodegenerative disorders, including MS.

The invention further relates to a compound comprising an FKBP inhibitor covalently linked to a cyclosporine for use in the treatment of a neurodegenerative disorder. Another aspect of the invention is a method of treating a neurodegenerative disorder, comprising administering to an individual in need thereof a therapeutically effective amount of a compound comprising an FKBP inhibitor covalently linked to a cyclosporine.

The neurodegenerative disorders that may be treated include Alzheimer's disease and other dementias, amyotrophic lateral sclerosis and other forms of motor neuron disease, Parkinson's disease, Huntington's disease, cerebral ischemia, neurological deficits associated with stroke and cerebral ischemia, all forms of degenerative disease affecting the central or peripheral nervous system (e.g. cerebellar-brainstem atrophies, syndromes of progressive ataxias), all forms of muscular dystrophy, progressive muscular atrophies, progressive bulbar muscular atrophy, physical or traumatic damage to the central or peripheral nervous system (e.g. spinal cord), herniated, ruptured or prolapsed intervertebrae disc syndromes, cervical spondylosis, plexus disorders, thoracic outlet syndromes, all forms of peripheral neuropathy (both diabetic and non-diabetic), trigeminal neuralgia, glossopharyngeal neuralgia, Bell's Palsy, all forms of auto-immune related disease resulting in damage of the central or peripheral nervous system (e.g. MS, myasthenia gravis, Guillain-Barre syndrome), AIDS related disorders of the nervous system, dapsone ticks, bulbar and retrobulbar affections of the optic nerve (e.g. retinopathies and retrobulbar neuritis), hearing disorders such as tinnitus, and prion diseases. As used herein, the term "cerebral ischemia" refers to a condition, wherein cerebral blood flow to a region of the brain is impeded or blocked, either temporarily, as in vasospasm or transient ischemic attack, or permanently, as in thrombotic occlusion. The affected region is deprived of oxygen and nutrients as a consequence of the ischemic event. This deprivation typically leads to the injuries of infarction in the region affected. Ischemia may also occur in the brain during a thromboembolic stroke, hemorrhagic stroke, cerebral vasospasm, head trauma/traumatic brain injury, cardiac arrest, severe blood loss due to injury or internal hemorrhage and other similar conditions that disrupt normal blood flow. It may also occur after a head trauma, since the pressure caused by edema presses against and flattens the arteries and veins inside the brain, thereby reducing their ability to carry blood through the brain. Cerebral ischemia may also occur as a result of macro- or micro-emboli and/or subsequent to cardiopulmonary or coronary artery bypass surgery.

Preferably, the present substances can be used for treating Alzheimer's disease or another dementia, Parkinson's disease, or MS. Most preferably, the disease to be treated is MS. The MS type which is treated may be relapsing remitting MS, secondary progressive MS, primary progressive MS, and/or progressive relapsing MS.

The substances of the invention can be administered alone but will generally be administered in admixture with a suitable pharmaceutical excipient diluent or carrier selected with regard to the intended route of administration and standard pharmaceutical practice. For example, the substances of the invention can be administered orally or sublingually in the form of tablets, capsules, ovules, elixirs, solutions or suspensions. The substances of the invention can also be injected parenterally, for example, intravenously, intraperitoneally, intrathecally, intraventricularly, intrasternally, intracranially, intramuscularly or subcutaneously, or they may be administered by infusion techniques. The substances of the invention can also be administered intranasally or by inhalation and are conveniently delivered in the form of a dry powder inhaler or an aerosol spray presentation from a pressurised container or a nebuliser with the use of a suitable propellant.

### BRIEF DESCRIPTION OF THE FIGURES

**Figure 1****.** Determination of hFKBP12 (black)-and hCypA (gray) inhibitory capacity of compound 7-10. The IC₅₀-values were measured via a protease coupled assay with a *h*FKBP12 concentration of 15 nM and a *h*CypA concentration of 5 nM. In the case of *h*FKBP12 the substrate suc-Ala-Phe-Pro-Phe-pNA and for *h*CypA suc-Ala-Ala-Pro-Phe-pNA was used.
**Figure 2****.** /*C*₅₀-values for the inhibition of the PPlase activity of FKBP12.6 (black), FKBP13 (gray) and FKBP52 (dashed) by compounds **7-10**
**Figure 3****.** /*C*₅₀-values for the inhibition of the PPlase activity of CypB (black), CypE (gray) and CypF (dashed) by compounds **7-10**
**Figure 4****.** /*C*₅₀-values for the inhibition of the PPlase activity of CypA by compounds **7-10** (black) and by compounds **7-10** complexed with FKBP12 (gray)
**Figure 5****.** /*C*₅₀-values for the inhibition of the PPlase activity of FKBP12 by compounds **7-10** (black) and by compounds **7-10** complexed with CypA (gray)
**Figure 6****.** Calcineurin inhibition activity of compounds **7-10** in complex with *h*FKBP12 (gray), *h*CypA (black) and *h*FKBP12+*h*CypA (dark gray). Concentration of 15 µM was used for *h*FKBP12 and *h*CypA. For complexation of compounds with *h*FKBP12 and *h*CypA simultaneously, 15 µM of each Immunophillin was used also.
**Figure 7****.** /C₅₀-values for the inhibition of the NFAT reporter gene activity by compounds **7-10**
**Figure 8****.** Effect of the bifunctional FKBP/cyclophilin inhibitor MT_11.14, consisting of FK506, the covalent linker diaminopropane and [D-Ser]⁸CsA], on clinical score of mice with experimental autoimmune encephalomyelits (EAE) as an animal disease model of Multiple Sclerosis.
**Figure 9****.** Determination of NFAT inhibitory capacity by compound MT_11.14 (gray) and FK506 and [D-Ser]⁸CsA] as control (dashed)
**Figure 10****.** Calcineurin inhibition activity of compound MT_11.14 in complex with hFKBP12, *h*CypA and *h*FKBP12+hCypA (gray) and FK506 and [D-Ser]⁸CsA] as control (dashed).Concentration of 15 µM was used for *h*FKBP12 and *h*CypA. For complexation of compounds with hFKBP12 and *h*CypA simultaneously, 15 µM of each Immunophillin was used also.
**Figure 11****.** Inhibition of the PPlase activity of FKBP12 and CypA by compound MT_11.14 (gray) and by FK506 and [D-Ser]⁸CsA] as control (dashed).
**Figure 12****.** /C₅₀-values for the inhibition of the PPlase activity of FKBP12.6, FKBP13 and FKBP52 (gray) by compound MT_11.14 in comparison with the PPlase inhibitory Cypacity of FK506]
**Figure 13****.** /C₅₀-values for the inhibition of the PPlase activity of CypB,CypE, CypF and CypD (gray) by compound MT_11.14 in comparison with the PPlase inhibitory Cypacity of [D-Ser]⁸CsA].

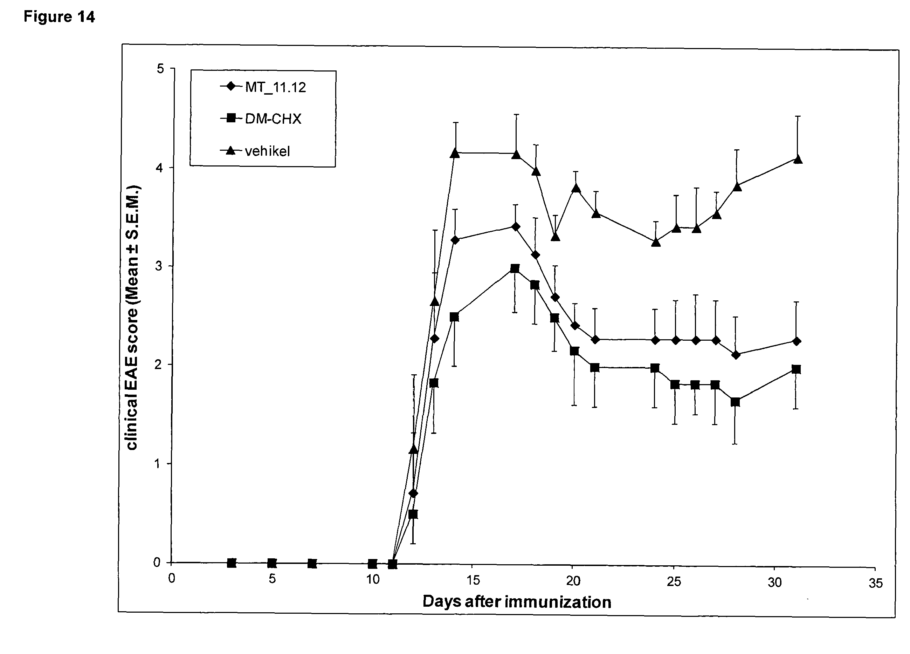

**Figure 14****.** Effect of the bifunctional FKBP/cyclophilin inhibitor MT_11.12, consisting of CHX covalently linked with [D-Ser]⁸CsA] by 2,2'-(Ethylendioxy)-diethylamine, on clinical score of mice with experimental autoimmune encephalomyelits (EAE) as an animal disease model of MS..

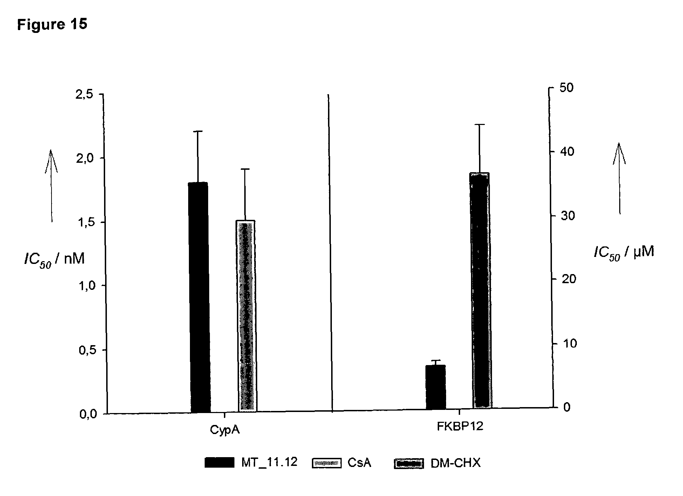

**Figure 15****.** Determination of CypA and FKBP12 inhibitory capacity by compound MT_11.14 (red) and [D-Ser]⁸CsA] (gray) and DM-CHX (dark gray) as control

### EXAMPLE 1

The starting compounds FK506 and CsA were modified by introducing an additional carboxyl group to crosslink both PPlase-inhibitors via diaminoalkyl spacers of different lengths. The following compounds were synthesized (for details, see experimental section *infra):*

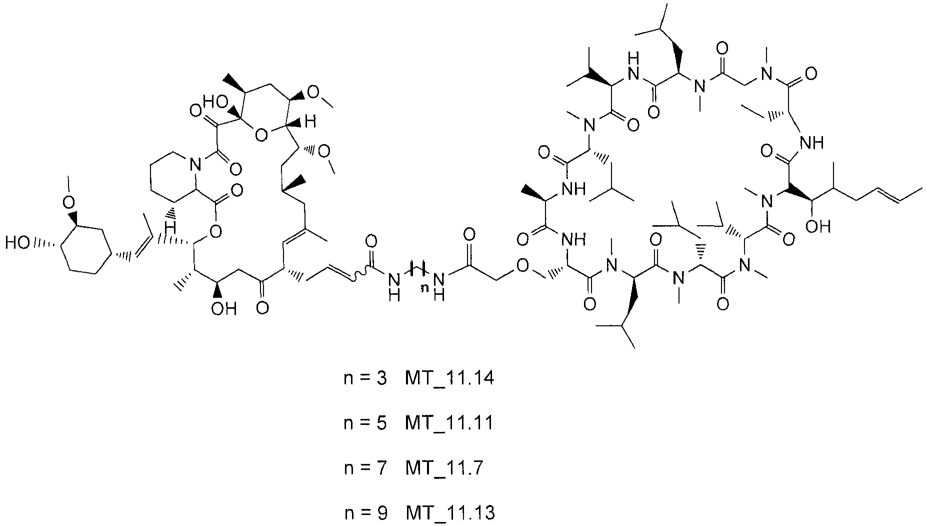

At FK506 the carboxyl group is introduced at carbon atom 40 which is localized in the CaN-binding domain. The carboxylic acid function of [O-Carboxymethyl-D-Ser⁸]-CsA (2) is attached to the amino acid residue 8 which is localized between the Cyclophilin and Calcineurin binding domain.

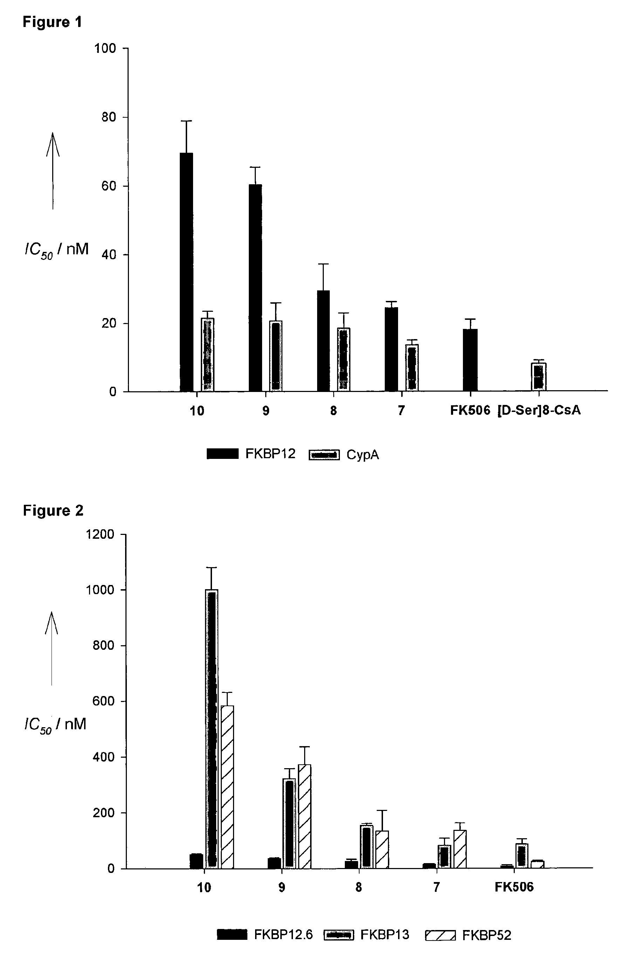

FK506 and [D-Ser⁸]-CsA are potent inhibitors of the peptidyl-prolyl-*cis*/*trans*-isomerases *h*FKBP12 and *h*CypA. After incubation with recombinant *h*FKBP12, **7-10** inihibited PPlase activity in low nanomolar range (Figure 1). Interestingly, the PPlase inhibitory properties depend on spacer length. The derivative with the shortest spacer causes the highest PPlase inhibitory activity. Under the experimental conditions used here, **7** and FK506 inhibited *h*FKBP12 with similar /C₅₀-values of (24.4±1.9 nM) and (18.1±3.0 nM) respectivly. In analogy *h*CypA is inhibited by 7 and [D-Ser⁸]-CsA /C₅₀-values of (14.2±1.7 nM) and (8.3±1.4 nM) respectively.

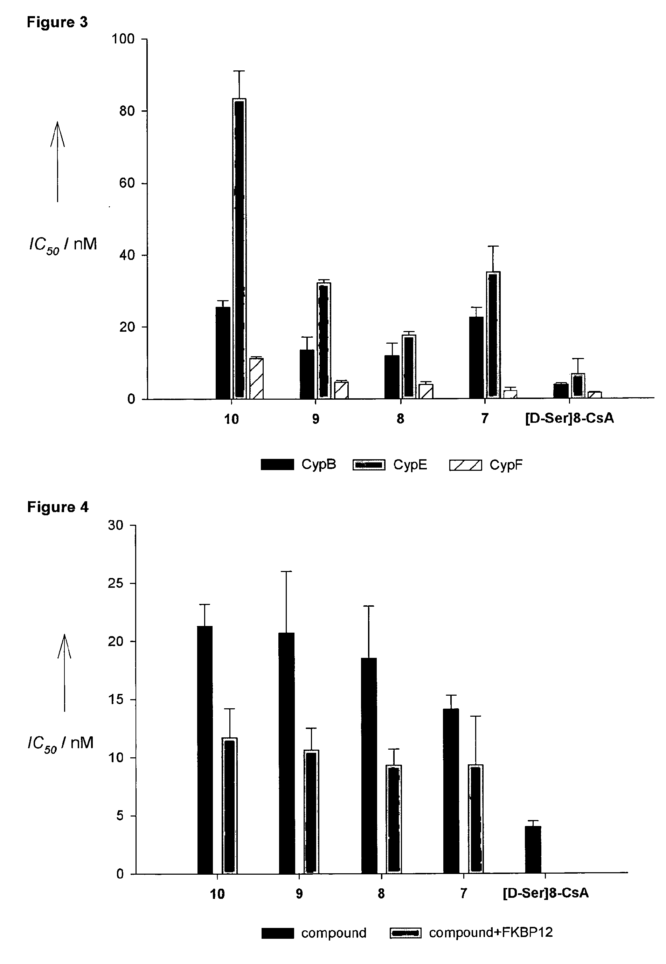

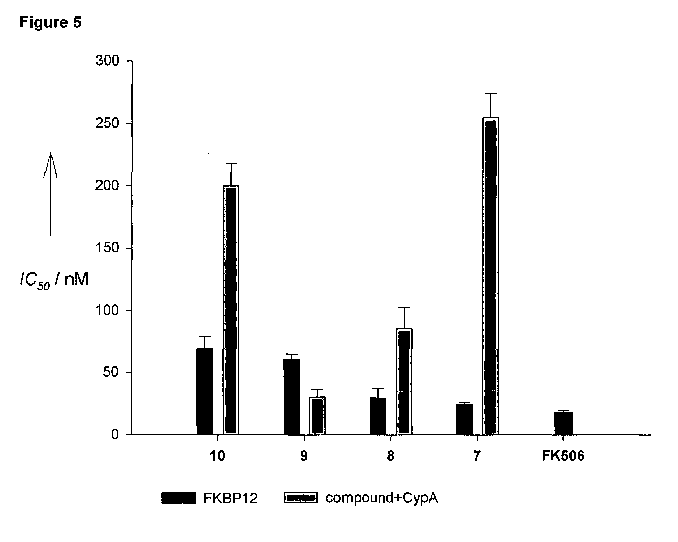

To investigate whether this phenomenon is limited to *h*FKBP12 and *h*CypA, other recombinant *h*FKBP, *h*FKBP 12.6, 13, 52) and *h*cyp (*h*CypB, E, F) were investigated as well. Beside *h*FKBP12 all other studied *h*FKBP show a dependency of the /*C*₅₀-values in a spacer length dependent manner (Figure 2). It became apparent that the Inhibition of the *h*FKBP decrease with increasing length of the spacer. Among the *H*FKBP, the *h*FKBP12.6 exhibited the highest affinity for the bifunctional compounds. Regarding the investigated *h*Cyp, a spacer length dependency was observed, too (Figure 3). Among the regarded Cyclophilins, *h*CypF is inhibited with the lowest /*C*₅₀-value by all compounds. In a further experiment **7-10** were pre-incubated with *h*FKBP12, and the *h*CypA inhibitory activity of these complexes was measured. As it can be seen in Figure 4, the *h*CypA inhibitory activity increases through pre-incubation with *h*FKBP12. In comparison with the non *h*FKBP12-complexed the *h*FKBP12-complexed compounds show two fold lower /*C*₅₀-values. These findings can be explained by the two facts. First, the bound *h*FKBP12 kept the bifunctional molecule in a conformation which is favored for *h*CypA binding. Second, the FK506 moiety of the bifunctional compounds which is complexed with *h*FKBP12 established a new surface. This *h*FKBP12/ FK506 surface interacts with *h*CypA in an allosteric way. This proposal is guided by the fact that the /C₅₀-values decrease with approximating FK506 and [D-Ser⁸]-CsA moieties. It behaves inverted when 7-10 are pre-incubated with *h*CypA and the *h*FKBP12 inhibition is measured (Figure 5). If the moieties (FK506 and [D-Ser⁸]-CsA) are in close proximity the ability of FKBP12 binding is suppressed due to the steric hindrance of CypA.

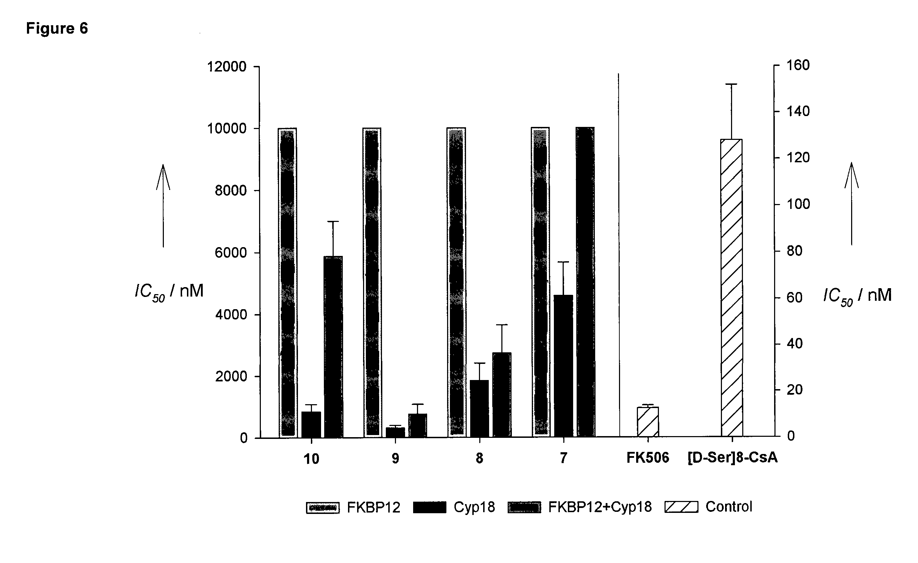

To ensure CaN inhibitory capacity of the compounds 7-10, an in vitro malachite green based assay was performed. For this purpose the CaN inhibitory activity of the formed *h*FKBP12/ compound, *h*CypA/ compound and *h*FKBP12/ compound/ *h*CypA complexes was determined. As shown in Figure 6, there is no inhibition of CaN up to a concentration of 10 µM if the bifunctional molecules are in complex with *h*FKBP12. In contrast, there is a considerably inhibition when compounds are complexed with *h*CypA only. These results indicate that the calcineurin inhibition is caused by the *h*CypA/ [D-Ser⁸]-CsA complex exclusively. Whereas the derivatives **7** and **8** display /*C*₅₀-values in lower micromolar range, **9** and **10** inhibit CaN in nanomolar concentrations. These data suggest that an elongation of the spacer will increase the ability of CaN inhibition. Surprisingly, comparison of **9** and **10** shows a decrease in inhibition. A possible explanation is, that spacers with more than 7 methylene groups will tend to self-assambly, thereby hindering the binding of calcineurin to the *h*CypA/ [D-Ser⁸]-CsA complex. The same order of inhibition capacities were observed when measuring the CaN-inhibition of **7-10** in presence of both, *h*FKBP12 and *h*CypA. Compound **9** showed the highest CaN-inhibition with an /*C*₅₀-value of 758.4 ± 312.8 nM while **7** showed no reduction of calcineurin activity up to 10 µM.

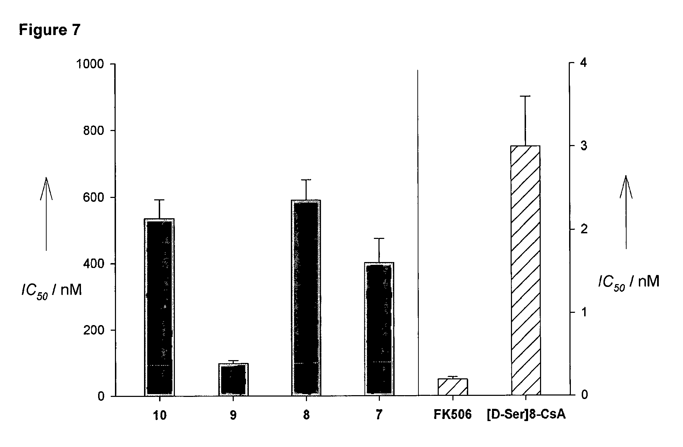

Next an NFAT-reporter gene assay was performed to establish the immunosuppessive properties of the compounds in vivo (Figure 7). Contrary to the CaN-assay, 7 displayed a considerable inhibitory activity with an /*C*₅₀-value of (402.4 ± 72.2 nM). Guided by the fact that *h*CypA is present in an excess in the cell compared to *h*FKBP12, it was assumed that the *h*CypA/ [D-Ser⁸]-CsA complex is formed primarily. The steric hindrance of the established *h*CypA/ [D-Ser⁸]-CsA complex in combination with the short spacer avoid the binding of *h*FKBP12, as observed by the PPlase measurement mentioned above. As a consequence the accessibility for calcineurin increases to bind to the *h*CypA/ [D-Ser⁸]-CsA complex. Herefrom results the immunosuppressive activity of **7.** As expected **8** showed lower NFAT inhibitory activity compared to **7.** The sterical hindrance of the *h*CypA/ [D-Ser⁸]-CsA complex became less distinct due to the longer spacer, so the ternary complex will be formed easier. The bound *h*FKBP12 interferes now on with the binding of CaN to the *h*CypA/ [D-Ser⁸]-CsA complex, resulting in a lower NFAT inhibitory capacity. Further elongation of the spacer **(9)** causes an increase of NFAT inhibitory activity. FK506 and [D-Ser⁸]-CsA are sufficiently far away from each other so that *h*FKBP12 and *h*CypA binding to the bifunctional compound. In the case of compound **9** *h*CypA does not interfere with *h*FKBP12 binding. Moreover, *h*FKBP12 does not disturb the binding of CaN to the ternary complex. This results in the lowest /C₅₀-value of (98.2 ± 9.6 nM) in the series of compounds. Comparison of **9** and **10** showed a decrease of NFAT inhibition capacity. This phenomenon is due to self assembly of the longer alkyl chain as already described.

### Experimental Section

### Materials

All chemicals, unless otherwise noted, were purchased from Sigma-Aldrich and were of reagent grade. Trifluoroacetic acid (TFA) and α-chymotrypsin were obtained from Merck. FK506 and Cyclosporine A (CsA) was obtained from LC Laboratories. Acetonitrile (ACN) HPLC gradient grade was purchased from Fisher Scientific. RPMI 1640 Medium, glutamine were obtained from PAA, Germany.

### Instrumentation

The preparative HPLC Sykam contained two S1021 pumps, a controller S2001, an Abimed 118 UV/VIS detector and reverse phase columns SP250x25 mm Hybar® C18, SP 250x21 mm Nucleosil C8 and 250x10 mm Interchrom C18. Solvent A: 0.05% TFA/H₂O. Solvent B: 0.05% TFA/ACN. The analytical HPLC- Dionex contained an ASI-100 autosampler, a P-680 pump, a PDA-100 photodiode array detector, a TCC-100 thermostated column compartment, the Chromeleon software program and Vydac® C18, 5µm, 4.6 x 250mm column. The same solvent system used for preparative HPLC was used for analytical HPLC. Demineralized water was additionally purified with a Millipore water purification system. ESI-spectra were recorded with an ESQUIRE-LC ion-trap mass spectrometer equipped with an ESI source (Bruker Daltronik, Bremen, Germany). Samples were injected with a syringe pump (Cole/Parmer Instrument Company). MALDI-ToF mass spectra were obtained with a Bruker REFLEX mass spectrometer (Bruker Daltronik, Bremen, Germany), using α-cyano-4-hydroxycinnamic acid as a matrix.

### Calcineurin activity measurement

Calcineurin activity was measured using a malachite green based assay. A 19-residue peptide of a partial sequence of the RII subunit of the bovine PKA (RII phosphopeptide) was used as substrate. For determination of indirect CaN inhibition by CsA derivative /cylcophilin 18 (15 µM) or FK506 derivative / FKBP12 (15 µM) or derivative /Cyp18wt /FKBP12 (15 µM) 25 nM - 10 µM of the CsA or FK506 derivatives, 1 U / µL CaN, 420 nM calmodulin were perincubated at 30 °C for 15 minutes in a 384-well plate. Dephosphorylation reaction was started by addition of RII phosphopeptide. After dephosphorylation for 30 minutes at 30 °C, malachite green reagent was added and absorption was measured during the next 15 to 20 minutes (PerkinElmer EnVision 2103 Multilabel reader). Activity is shown as the mean of triplicates ± S.D., expressed as percentages of calcineurin activity relative to an inhibitor-free control (100 %) and a calcineurin free control (0 %).

### NFAT reporter gene assay

Jurkat T cells were electroporated according to an Amaxa protocol (Amaxa, Cologne, Germany) with NFAT-luciferase reporter gene plasmids (1 - 1.5 µg) (Promega, Mannheim, Germany). The transfected cells were cultured in RPMI 1640 with 10 % FCS and 2 mM L-glutamine for 16 hours in a humidified chamber with 5 % CO₂ at 37 °C. Aliquots of electroporated cells were preincubated with the inhibitors or 0.5% DMSO (control) for 30 min, followed by stimulation with 10 ng / mL PMA + 1 µg / mL ionomycin or 100 ng / mL TNF-alpha for 5 h. After cell lysis, the level of the extracted luciferase was determined by bioluminescence measurement using the luciferase assay system (Promega).

### Cell culture

Jurkat T cells were cultured in RPMI 1640 medium with 10 % fetal bovine serum (FBS) and 2 mM stable L-glutamine in a humidified chamber with 5 % CO₂ at 37 °C.

### Protease-Coupled PPlase Assay

The PPlase activity of *h*FKBP12 and *h*CypA was determined by using the protease-coupled PPlase assay as described by Fischer, H. Bang, B. Ludwig, K. Mann, J. Hacker, Mol. Microbiol. 1992, 6, 1375-1383. To study the effect of compounds **7-10** on PPlase activity, 100 nM of each *h*FKBP and 5 nM of each *h*Cyp, respectively and 40 µM peptide substrate were preincubated in the presence or absence of various inhibitor concentrations for 6 min at 10 °C in 35mM HEPES/NaCl buffer (pH 7.5). Then reaction was started by addition of 100 µg/mL of the isomer specific protease chymotrypsin, and the release of 4-nitroaniline was monitored by measuring the absorbance at 390 nm. To achieve better comparability of compounds **7-10** effects on PPlase activity, the concentrations of FKBP and Cyp were kept constant at 100 nM and 5 nM, respectively. Since tested PPlases vary in there catalytic efficiencies, different substrates has to be used to ensure equal rate constants of catalyzed *cis*/*trans* isomerization (Table 1). Substrates possess the general form Suc-Ala-*Xaa*-Pro-Phe-pNA.

**Table 1. Substrates for the respective PPlases**

| FKBP | Suc-AXPF-pNA *X* | Cyclophillin | Suc-AXPF-pNA *X* |
|---|---|---|---|
| 12 | Lys | A | Phe |
| 12.6 | Ile | B | Phe |
| 13 | Phe | E | Gly |
| 52 | Lys | F | Gly |

The preparation of the bifunctional compounds commenced with an olefin metathesis between FK506 and acryclic acid to generate **1.** For the syntheses of **3-6,** derivative **1** was pre-activated by HATU and coupled as an amide to the respective amine. Finally the [O-Carboxymethyl-D-Ser⁸]-CsA was attached to compounds **3-6** in the manner mentioned before to provide **7-10.**

### Synthesis of compound (1):

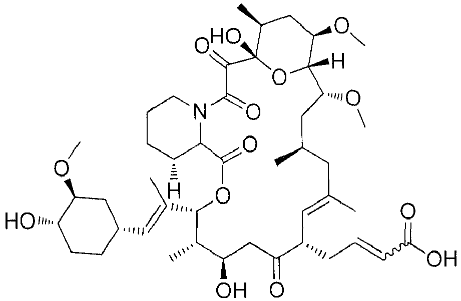

To a solution of FK506 (100 mg; 124.4 µmol) in 6 ml of 1,2-Dichlorbenzene Hoveyda-Grubbs catalyst 2^{nd} generation (7.8 mg, 12.4 µmol) and acrylic acid (177 µl; 2.6 mmol) was placed in a microwave vial. The mixture was irradiated at 200 W and 150 °C for 20 min. The solvent was evaporated and the crude product was purified via preparative HPLC. First a RP C8 column (Nucleosil 250 x 25) was used with a linear gradient from 20 % - 90 % B in 60 min with a flow rate of 17 ml/min. The product was lyophilized and purified a second time using a RP C18 column (Hybar 250 x 25) with a linear gradient from 30 % - 80 % B in 40 min with a flow rate of 17 ml/min. Twice purification steps were used to remove the catalyst. The fractions were collected, evaporated and lyophilized to obtain the product as a whit solid with a yield of 79 mg (75.2 %), [M+H⁺]⁺_{calc.} = 847.5, [M⁺H⁺] ⁺_{found} = 848.6.

See also:
P.S. Marinec, C.G. Evans, G.S. Gibbons, M. A. Tarnowski, D.L. Overbeek, J.E. Gestwicki, Bioorg.Med. Chem. 2009, 17, 5763-5768.

### Synthesis of compound (2):

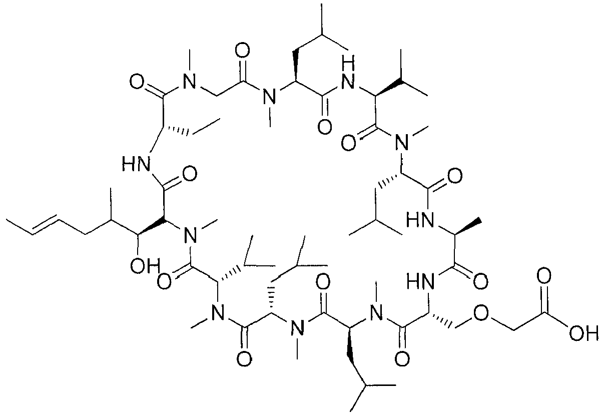

Compound (2) was prepared as described in M. K. Eberle, P. Hiestand, A-M. Jutzi-Eme, F. Nuninger, H. R. Zihlmann, J. Med. Chem. 1995, 38, 1853-1864.

### General Procedure 1 for synthesis of compound 3-6:

Compound 1 (10 mg; 11.8 µmol), *N*,*N*,*N*',*N'-*tetramethyl-*O*-(7-azabenzotriazol-1-yl)uronium hexafluorophosphate (4.5 mg; 11.8 µmol), N-ethyldiisopropylamine (4.21 µl, 23.6 µmol) were dissolved in 4 ml DMF and preactivated for 5 min. The respective diamine (23.6 µmol) was added and the mixture was stirred for further 2 h at room temperature. The solvent was evaporated and the crude product was purified via preparative HPLC using a RP C18 column (Interchrom 250 x 10). A linear gradient from 30 % - 80 % B in 40 min with a flow rate of 5 ml/min was used.

### Synthesis of compound (3):

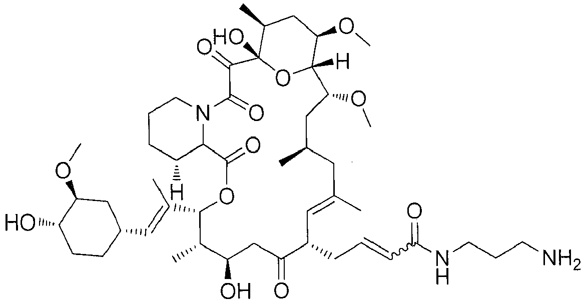

According to general procedure 1 described above, compound 1 was react with 1,3-Diaminopropane (2.0 µl; 23.6 µmol). After purification, the product was achieved as a white solid with a yield of 7.5 mg (70.7 %), [M+H⁺] ⁺_{calc} = 904.5, [M+H⁺] ⁺_{found} = 904.3.

### Synthesis of compound (4):

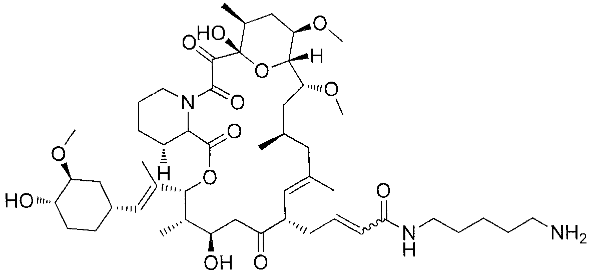

Compound 1 was react with 1,5-Diaminopropane (2.8 µl; 23.6 µmol) according to general procedure 1 . After purification, the product was achieved as a white solid with a yield of 5.4 mg (49.5 %), [M+H⁺]⁺_{calc.} = 932.2, [M+H⁺] ⁺_{found} = 932.4.

### Synthesis of compound (5):

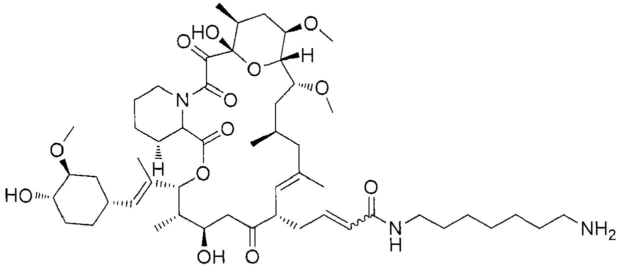

Compound 1 was react with 1,7-Diaminopropane (3.0 mg; 23.6 µmol) according to general procedure 1. After purification, the product was achieved as a white solid with a yield of 6.2 mg (54.8 %), [M⁺H⁺] ⁺_{calc.} = 960.2, [M⁺H⁺] ⁺_{found} = 960.5.

### Synthesis of compound (6):

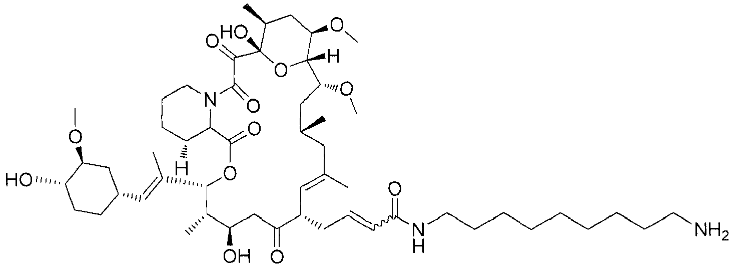

Compound 1 was react with 1,9-Diaminopropane (7.0 mg; 23.6 µmol) according to general procedure 1 . After purification, the product was achieved as a white solid with a yield of 7.1 mg (57.7 %), [M⁺H⁺]⁺_{calc.} = 1044.4, [M⁺H⁺]⁺_{found} = 1044.8.

### General Procedure 2 for synthesis of compound 7-10:

[*O*-Carboxymethyl D-serine]⁸ -CsA (11.6 mg; 9.2 µmol mmol), synthesized as previously published^{[10]}, was preactivated with *N*,*N*,*N*',*N*'-tetramethyl-*O*-(7-azabenzotriazol-1-yl)uronium hexafluorophosphate (3.5 mg; 9.2 µmol) and *N*-ethyldiisopropylamine (5.0 µl, 28.1 µmol in 4 ml DMF for 5 min. The respective compound **2-5** (11.0 µmol) was added and the mixture was stirred for further 2 h at room temperature. The solvent was evaporated and the crude product was purified via preparative HPLC using a RP C18 column (Hybar 250 x 25). A linear gradient from 40 % - 90 % B in 40 min with a flow rate of 17 ml/min was used.

### Synthesis of compound (7):

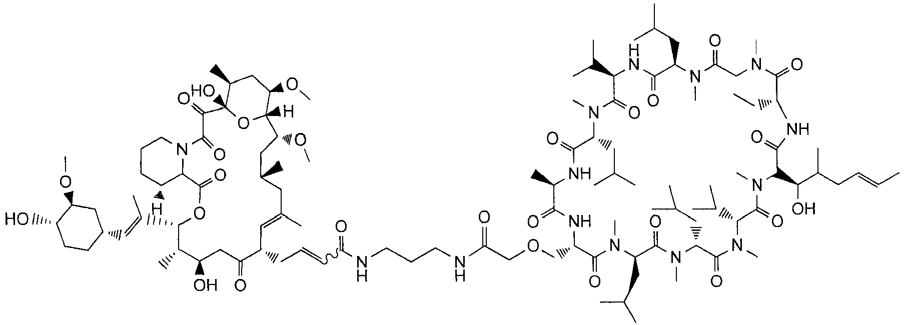

According to general procedure 2, [*O*-Carboxymethyl D-serine]⁸ -CsA was react with compound 3 (9.9 mg; 11.0 µmol). After purification, the product was achieved as a white solid with a yield of 10.2 mg (51.2 %), [M+H⁺]⁺_{calc.} = 2162.4, [M+H⁺] ⁺_{found} = 2162.6.

### Synthesis of compound (8):

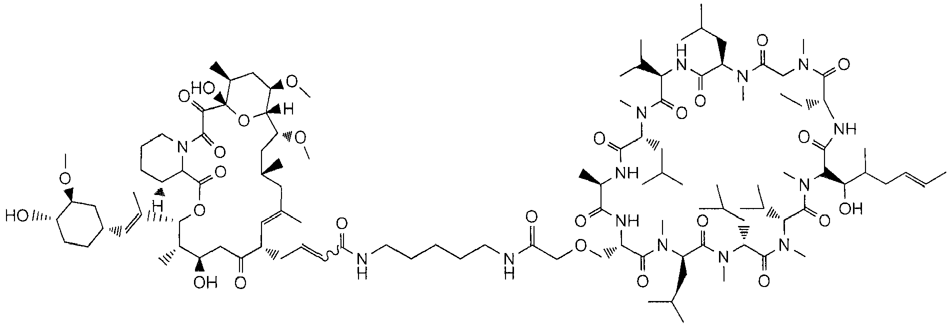

According to general procedure 2, [O-Carboxymethyl D-serine]⁸ -CsA was react with compound 4 (10.2 mg; 11.0 µmol). After purification, the product was achieved as a white solid with a yield of 15.4 mg (76.6 %), [M+H⁺] ⁺_{calc.} = 2190.4, [M+H⁺] ⁺_{found} = 2190.2.

### Synthesis of compound (9):

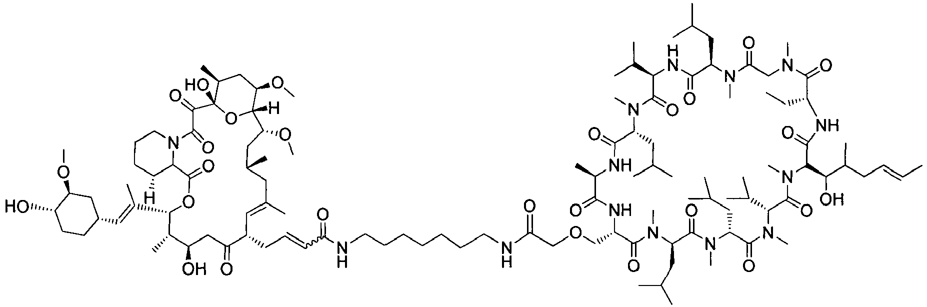

According to general procedure 2, [O-Carboxymethyl D-serine]⁸ -CsA was react with compound 5 (10.5 mg; 11.0 µmol). After purification, the product was achieved as a white solid with a yield of 13.1 mg (64.2 %), [M+H⁺]⁺_{calc.} = 2218.5, [M+H⁺]⁺_{found} = 2218.2.

### Synthesis of compound (10):

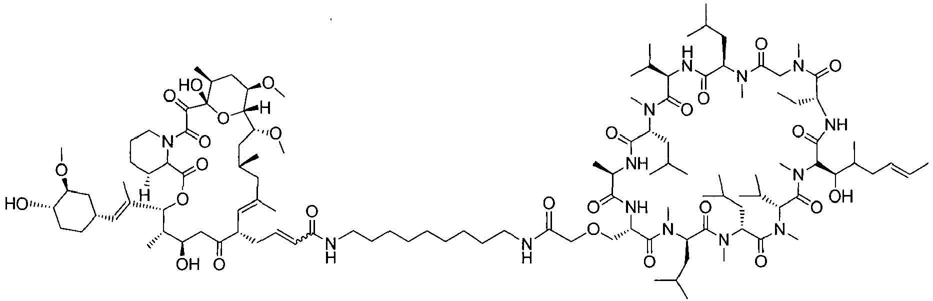

According to general procedure 2, [O-Carboxymethyl D-serine]⁸-CsA was react with compound 6 (11.4 mg; 11.0 µmol). After purification, the product was achieved as a white solid with a yield of 11.7 mg (56.8 %), [M+H⁺]⁺_{calc.} = 2246.9, [M+H⁺]⁺_{found} = 2246.6.

### EXAMPLE 2

### Effect of the bifunctional FKBP/cyclophilin inhibitor MT_11.14, consisting of FK506, the covalent linker diaminopropane and [D-Ser]⁸CsA], on clinical score of mice with experimental autoimmune encephalomyelits (EAE) as an animal disease model of Multiple Sclerosis.

### Model

EAE was induced by immunization of SJL/J mice with proteolipid protein (PLP) peptide 139-151. Female SJL/J mice received a subcutaneous (s.c.) injection (100 µl) of 200 µg PLP in 0.1 ml PBS emulsified in an equal volume of complete Freund's adjuvant (CFA), containing 6 mg/ml mycobacterium tuberculosis H37Ra.

Immunization of SJL/L mice with PLP results in the development of a remitting/relapsing form of EAE with clinical signs manifested as paralysis typically 10-14 days after immunization.

Mice were scored daily for clinical signs of EAE according to the following increasing severity scale: 0, no abnormality; 1, limp tail and animal cannot be flipped easily on its back; 2, limp tail and animal can be flipped easily on its back; 3, partial hind-limp weakness; 4, hind-limp weakness; 5, partial hind-limp paralysis; 6, hind limp paralysis; 7, hind limp paralysis with partial fore-limp weakness; 8, hind-limp and fore-limp paralysis; 9, moribund.

### Compounds, application and data analysis

The compound designated MT_11.14 had the following structure:

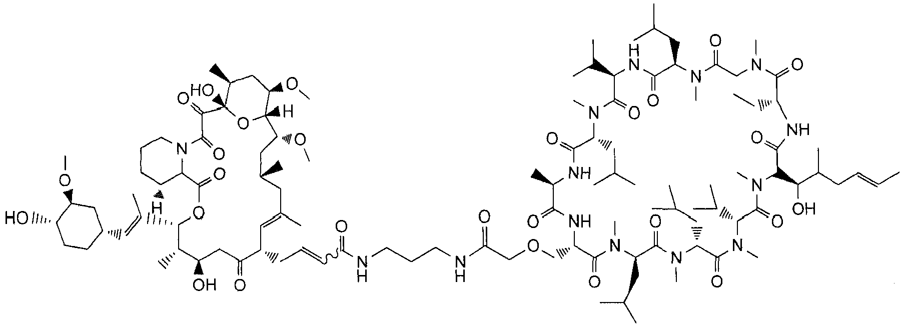

MT_11.14 (10 mg/kg/day) or a mixture of FK506 and CsA (not linked covalently; each compound 5 mg/kg/day) was dissolved in 0.9% saline containing 30% DMSO. The compounds were administered continuously for 24 consecutive days via mini-osmotic pumps which were implanted intraperitoneally into mice immediately after induction of EAE. Vehicle: 0.9% saline containing 30% DMSO.

Data are given as mean ± S.E.M. Statistical analysis was performed using Student's t-test. P ≤ 0.01 between drug and vehicle treatment. n = 5 - 8 animals per treatment group.

### Results

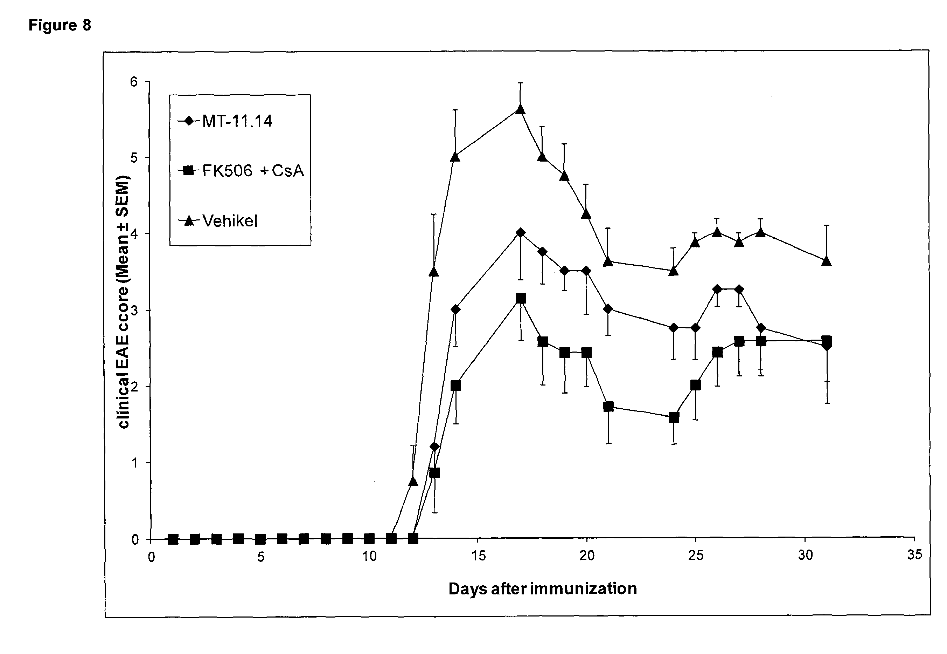

The results are depicted in Figure 8. Compared to vehicle-treated control animals, MT_11.14 caused a significant reduction of clinical EAE scores at days 17 and 31 post immunization by 30.5% ± 7.7% and 28.5% ± 6.8% , respectively (P ≤ 0.01). In parallel, the mixture of FK506 and CsA decreased the clinical EAE score by 44.6% ± 7.1% and 27.7% ± 6.5%, repectively, if compared to vehicle treatment (P ≤ 0.01).

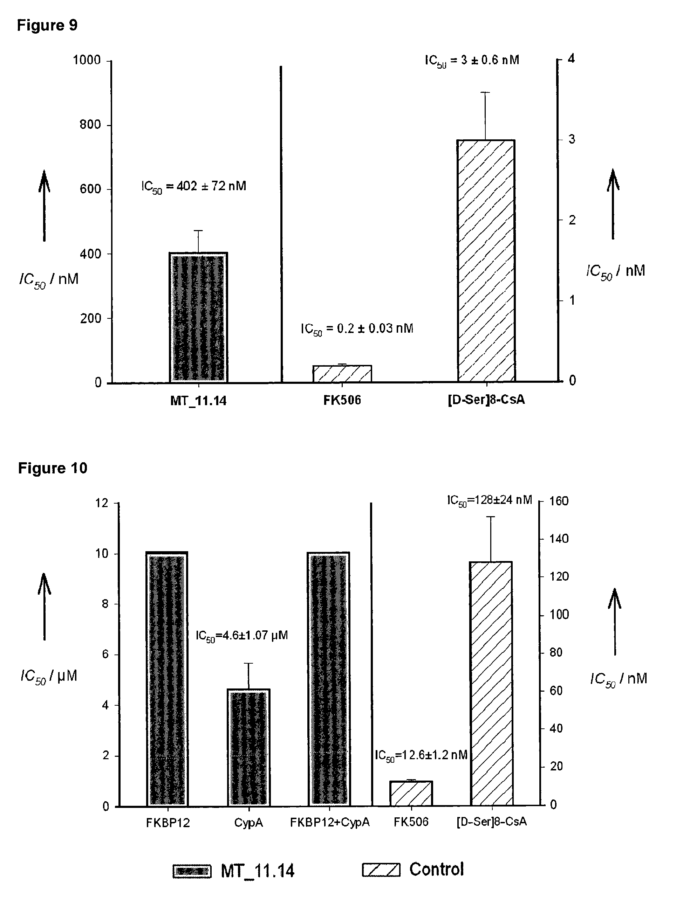

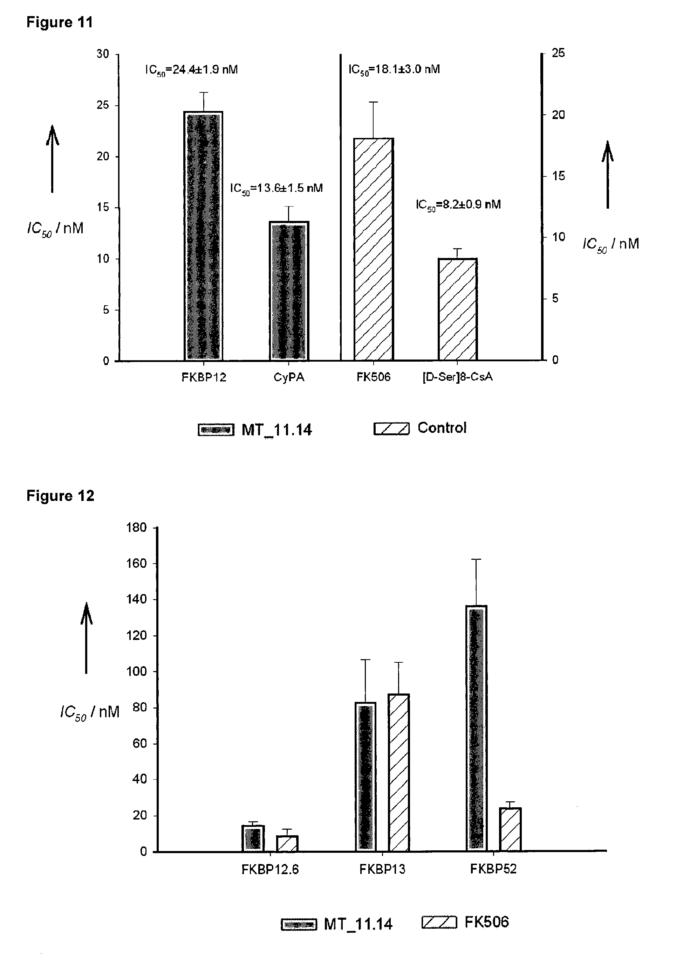

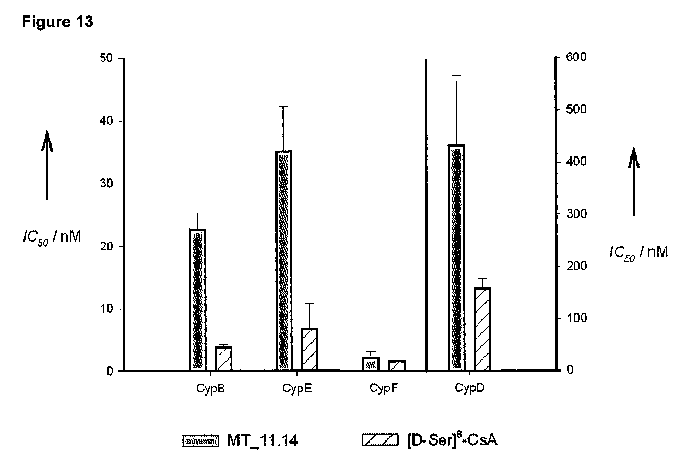

PPlase inhibitory activity, NFAT inhibitory activity and Calcineurin inhibitory activity of MT_11.14 (compound 7) were already determined in Example 1. The results for MT_11.14 are depicted in Figures 9-13.

### EXAMPLE 3

### Effect of the bifunctional FKBP/cyclophilin inhibitor MT_11.12, consisting of CHX covalently linked with [D-Ser]⁸CsA] by 2,2'-(Ethylendioxy)-diethylamine, on clinical score of mice with experimental autoimmune encephalomyelits (EAE) as an animal disease model of MS.

The compound MT_11.12 has the following structure:

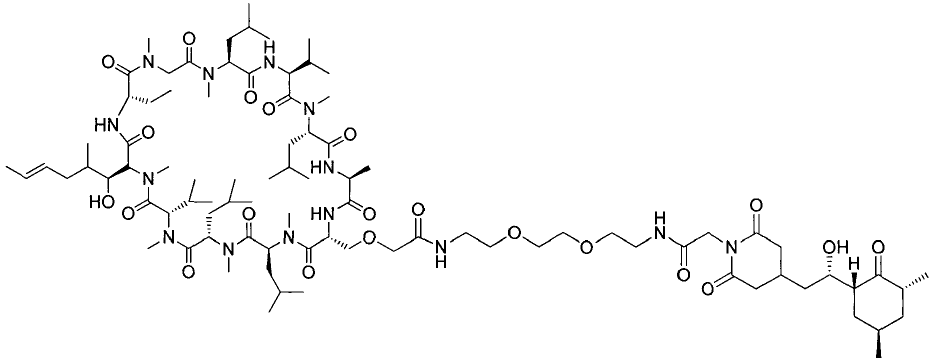

For experimental designs, see Example 2. MT_11.12 or DM-CHX were continuously administered from day 0 to day 21 post immunization. Compared to vehicle, MT_11.12-treated animals revealed a significant reduction of clinical EAE scores. DM-CHX was used as positive control. n = 8 - 10 SJL/J mice per treatment condition.

To prove the PPlase inhibitory capacity of MT_11.12, we have determined the IC₅₀-value of the inhibition of CypA and FKBP12. If we consider the inhibition of CypA, there is only a slight difference between the IC₅₀ value of MT_11.12 (1.8 ± 0.4 nM) and [D-Ser]⁸-CsA (1.5 ± 0.4 nM). Thus we can say that there is no steric hindrance of the FKBP inhibitor with respect to the binding of the [D-Ser]⁸-CsA residue to CypA. There is a remarkable difference when one considers the inhibition of FKBP12. The PPlase activity of FKBP12 was inhibited by MT_11.12 with an IC₅₀ value of 6.9 ± 0.7 nM while DM-CHX is inhibiting with an IC₅₀ value of 36.9 ± 17.1 nM. Therefore MT_11.12 inhibits the rotamase activity of FKBP12 five times better than DM-CHX.

## Claims

**1.** A compound comprising an FKBP inhibitor covalently linked to the amino acid residue at position 8 of a cyclosporine.

**2.** The compound of claim 1, represented by the structural formula Cs-L-F, wherein
Cs is the cyclosporine,
L is a linker, and
F is the FKBP inhibitor.

**3.** The compound of claim 1 or 2, wherein the cyclosporine is Cyclosporin A or a derivative thereof.

**4.** The compound of any one of claims 1 to 3, wherein the linker is an optionally substituted aliphatic, alicyclic, heteroaliphatic, heterocyclic, aryl, arylalkyl, or heteroaromatic group and consists of 5 to 300 atoms.

**5.** The compound of claim 4, wherein linker is a C₁₋₂₀, saturated or unsaturated, straight or branched, hydrocarbon chain, wherein one or more methylene units of the linker are optionally and independently replaced by phenylene, piperazinylene, piperidinylene, pyridinylene, morpholinylene, 1,4-dioxanylene, pyrrolylene, furanylene, thiophenylene, thiazolylene, oxazolylene, imidazolylene, -N=N-, -N(R)-, -N(R)C(O)N(R)-, -N(R)C(O)O-, -OC(O)N(R)-, -N(R)C(O)-, -C(O)N(R)-, -N(R)SO₂-, - SO₂N(R)-, -O-, -C(O)-, -OC(O)-, -C(O)O-, -S-, -SO-, -SO₂-, -C(=S)-, or -C(=NR)-; wherein R is selected from the group consisting of hydrogen and C₁₋₆ alkyl.

**6.** The compound of any one of the preceding claims, wherein the FKBP inhibitor is the compound FK506.

**7.** The compound of any one of claim 1 to 5, wherein the FKBP inhibitor is DM-CHX.

**8.** The compound of claim 6, which is

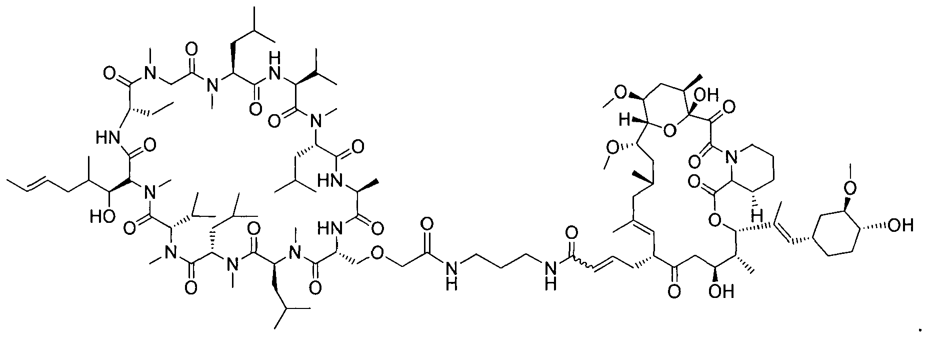

**9.** The compound of claim 7, which is

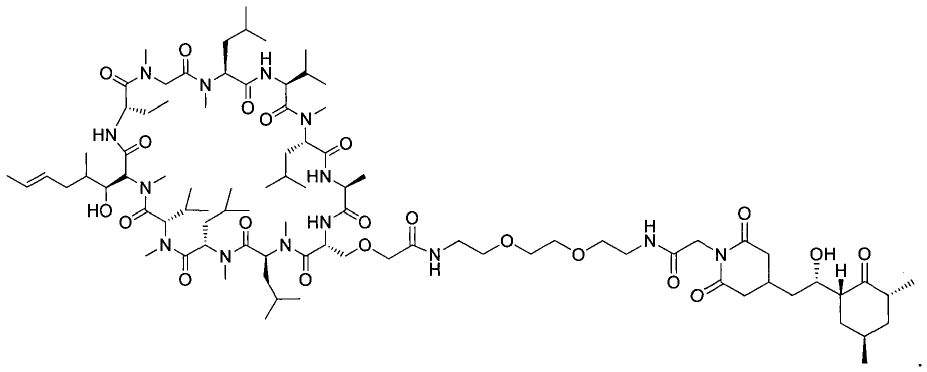

**10.** The compound of any one of the preceding claims which is capable of reducing the clinical signs of experimental autoimmune encephalomyelitis (EAE) in a mouse model.

**11.** The compound of any one of the preceding claims which is capable of inhibiting at least one FKBP and at least one cyclophilin.

**12.** The compound of any one of the preceding claims having a lower immunosuppressive effect than a mixture of the cyclosporine and the FKBP inhibitor, as determined in a calcineurin inhibition assay.

**14.** A compound comprising an FKBP inhibitor covalently linked to a cyclosporine for use in the treatment of a neurodegenerative disorder.

**15.** The compound for use according to claim 14, wherein said neurodegenerative disorder is selected from the group consisting of multiple sclerosis, Alzheimer's disease, Parkinson's disease, cerebral ischemia, Huntington's disease and amyotrophic lateral sclerosis.

**16.** The compound for use according to claim 14 or 15, which is a compound as defined in any one of claims 1 to 13.
